# EUROPÄISCHE PATENTSCHRIFT

(11) **EP 4 568 740 B1**
(45) Veröffentlichungstag und Bekanntmachung des Hinweises auf die Patenterteilung: **09.12.2026**
(21) Anmeldenummer: 24799139.1
(22) Anmeldetag: 25.10.2024
(51) Int. Cl.: A61H 39/00, A61N 1/05, A61N 1/36, A61N 1/378

(54) **NADELEINRICHTUNG ZUR PLATZIERUNG AN EINER OHRMUSCHEL**
NEEDLE DEVICE FOR PLACING ON AN AURICLE
DISPOSITIF D'AIGUILLE DESTINÉ À ÊTRE PLACÉ SUR LE PAVILLON DE L'OREILLE

(30) Priorität: 26.10.2023 DE 102023129520
(43) Veröffentlichungstag der Anmeldung: 18.06.2025
(60) Teilanmeldung: 26212005.8
(73) Patentinhaber: biolitec Holding GmbH & Co KG, 1030 Wien (AT)
(72) Erfinder: LANDL, Gabriel, 7222 Rohrbach (AT); NEUBERGER, Wolfgang, 1010 Wien (AT); WIELAND, Gerhard D., 07751 Jena (DE); WIEHE, Arno, 10777 Berlin (DE); SCHNEIDER, André, 51491 Overath (DE)
(74) Vertreter: Aurigium Leischner & Luthe
(86) Internationale Anmeldenummer: PCT/EP2024/080285
(87) Internationale Veröffentlichungsnummer: WO 2025/088153

(56) Entgegenhaltungen:
- EP-A1- 4 257 175
- WO-A1-2023/164132
- US-A1- 2018 064 603
- US-B2- 8 942 814

## Beschreibung

Der präsentierte Ansatz betrifft eine Nadeleinrichtung zur Platzierung an einer Ohrmuschel, eine Nervenstimulationsvorrichtung zum elektrischen Stimulieren zumindest eines Nervs und ein Verfahren zur Verwendung einer Nadeleinrichtung gemäß den Hauptansprüchen.

Neurostimulation, genauer gesagt Elektrostimulation peripherer Nerven, ist seit langem bekannt und kann bei einer Vielzahl von Erkrankungen eingesetzt werden, zum Beispiel zur Schmerzbehandlung oder Schmerzlinderung, bei peripheren arteriellen Gefäßerkrankungen, bei Entzündungen oder entzündlichen Zuständen oder bei Vorhofflimmern. Eine solche Elektrostimulation der peripheren Nerven kann mit verschiedenen Methoden durchgeführt werden:
Bei einer transkutanen Nervenstimulation (oder TENS für "transkutane elektrische Nervenstimulation") können Elektroden verwendet werden, die auf der Haut in einem schmerzhaften Bereich oder auf einer Nervenbahn angebracht werden, beispielsweise um elektrische Impulse zu senden.

Bei einer perkutanen Nervenstimulation (oder PENS für "perkutane elektrische Nervenstimulation") kann ein entsprechender Nerv oder können entsprechende Nerven direkter stimuliert werden. Dabei können die Elektroden entweder im Rahmen eines chirurgischen Eingriffs unter die Haut gelegt oder Elektroden zum Beispiel in Form von Akupunkturnadeln verwendet werden.

Bei TENS und/oder bei PENS können die von den Elektroden ausgesandten elektrischen Impulse zu einer Depolarisation am entsprechenden Nerv führen, wenn sich eine Stimulationselektrode nahe genug am Nerv befindet. Lokale Parästhesien entstehen üblicherweise an afferenten Nervenendigungen. Die Elektrode oder eine Injektionsnadel sollte nicht in direktem Kontakt mit dem Nerv stehen.

Insbesondere der Vagusnerv wird herkömmlicherweise als Zielnerv für die Elektrostimulation und/oder beispielsweise zur Verbesserung und/oder Behandlung verschiedener medizinischer und nichtmedizinischer Zustände ausgewählt. Der Vagusnerv, der zehnte und längste der Hirnnerven, ist ein wichtiger Teil des parasympathischen Nervensystems und kann als bidirektionale Verbindung zwischen Körper und Gehirn dienen. Der Vagusnerv verläuft vom Hirnstamm zu den proximalen zwei Dritteln des Dickdarms und innerviert auf diesem Weg mehrere thorakale und abdominale innere Organe. Bei dem Vagusnerv handelt sich um einen gemischten Nerv, der zu 20 Prozent aus efferenten und zu 80 Prozent aus afferenten Fasern besteht. Eine Stimulation dieses Nervs kann zum Beispiel über den aurikulären Ast des *Nervus vagus* erfolgen. Alternativ ist auch eine Stimulation des Vagusnervs im Halsbereich (zervikaler Ast) möglich.

Das Dokument US 2018/064603 A1 offenbart ein System und ein Verfahren zur aurikulären peripheren Nervenfeld-Stimulation.

Das Dokument WO 2023/164132 A1 offenbart ein aurikuläres nervenfeld-Stimulationsgerät und Verfahren, die dieses benutzen.

Das Dokument EP 4 257 175 Al offenbart ein Elektrodenarray und eine Elektrode für die periphere Nervenstimulation.

Der hier vorgestellte Ansatz stellt sich die Aufgabe, eine verbesserte Nadeleinrichtung zur Platzierung an einer Ohrmuschel, eine Platzierungseinheit, eine verbesserte Nervenstimulationsvorrichtung zum elektrischen Stimulieren zumindest eines Nervs und ein verbessertes Verfahren zur Verwendung einer Nadeleinrichtung gemäß den Hauptansprüchen zu schaffen. Vorteilhafte Ausgestaltungen und Weiterbildungen ergeben sich aus den Unteransprüchen und der nachfolgenden Beschreibung.

Gemäß dem hier präsentierten Ansatz wird diese Aufgabe durch eine verbesserte Nadeleinrichtung zur Platzierung an einer Ohrmuschel mit den Merkmalen des Hauptanspruchs gelöst. Vorteilhafte Ausgestaltungen und Weiterbildungen des präsentierten Ansatzes ergeben sich aus der Beschreibung und den nachfolgenden Unteransprüchen.

Zur Lösung der vorstehend genannten Aufgabe schafft der hier vorgeschlagene Ansatz eine Nadeleinrichtung, die ausgebildet ist, um an der Ohrmuschel und/oder am Ohrläppchen platziert oder platzierbar zu sein, wobei die Nadeleinrichtung eine Griffeinrichtung und/oder eine Platzierungseinrichtung zum Platzieren der Nadeleinrichtung an der Ohrmuschel und/oder am Ohrläppchen aufweist.

Unter einer Nadeleinrichtung kann allgemein ein technisches Bauelement verstanden werden, das zumindest eine Nadel oder auch mehrere Nadeln aufweist, die mit einer elektrischen Spannung beaufschlagt oder versetzt werden können, sodass über diese Nadel bzw. Nadeln ein elektrischer Impuls auf ein mit der Nadel bzw. den Nadeln verbundenes Körperteil übertragen werden kann. Diese (elektrischen) Impulse können durch die Impulseinrichtung bereitgestellt werden und an die mit der Impulseinrichtung koppelbare Nadeleinrichtung an das Ohr bzw. das Ohrläppchen übertragen werden.

Hierbei weist die Nadeleinrichtung, die auch als Nadeleinheit bezeichnet werden kann, einen Griff auf, der in der vorliegenden Beschreibung auch als Griffeinrichtung zum Platzieren der Nadeleinrichtung an der Ohrmuschel und/oder am Ohrläppchen bezeichnet werden kann. Alternativ oder zusätzlich kann eine Platzierungseinrichtung zum Platzieren der Nadeleinrichtung an der Ohrmuschel und/oder am Ohrläppchen verwendet werden. Unter Verwendung der Griffeinrichtung - alternativ Manipulator oder abnehmbarer Manipulator genannt - kann die Nadeleinrichtung durch einen Anwender oder durch medizinisches Personal einfach und steril an der Ohrmuschel und/oder am Ohrläppchen positioniert werden. Durch ein Abbrechen der Griffeinrichtung, die auch als Führungsstab bezeichnet werden kann, ist eine wiederholte, möglicherweise unsterile Anwendung der Nadeleinrichtung kaum möglich. Bei der Verwendung einer Platzierungseinrichtung kann die Nadeleinheit mit den Nadeln beispielsweise schnell, zerstörungsfrei und somit positionsstabil von einem Griffstück bzw. der Platzierungseinheit getrennt werden.

Der hier vorliegende Ansatz basiert auf der Erkenntnis, dass eine breite Anwendung herkömmlicher Technologie im medizinischen Bereich, aber auch im Bereich der Heimanwendung ermöglicht werden kann. Zu diesem Zweck ist es vorteilhaft, insbesondere eine Platzierung und/oder eine Installation der Elektroden am Ohr zu vereinfachen. Diese Vereinfachung kann dadurch erfolgen, dass die Nadeleinrichtung von der Impulseinrichtung getrennt wird oder trennbar ist und sich somit separat am Ohr bzw. Ohrläppchen befestigen lässt. Dies bietet den Vorteil, dass die Platzierung der Nadeleinrichtung ohne die hierzu störende größere Impulseinrichtung erfolgen kann, sodass nach erfolgter Anbringung der Nadeleinrichtung Platzierung eine elektrische Verbindung der Nadeleinrichtung mit der Impulseinrichtung möglich ist, ohne dass sich die Nadeln der Nadeleinrichtung von der gewünschten Position verschiebt und somit eine Fehlpositionierung verursacht.

Günstig ist eine Ausführungsform des hier vorgestellten Ansatzes, bei dem die Nadeleinrichtung eine Sollbruchstelle zum Trennen der Griffeinrichtung von der Nadeleinrichtung aufweist. Alternativ oder zusätzlich kann die Platzierungseinrichtung der Nadeneinrichtung als Pinzette und/oder als Klammer ausgebildet sein und/oder die Platzierungseinrichtung der Nadeneinrichtung ein Schnappverschlusselement und/oder ein Klickverschlusselement und/oder ein Magnetverschlusselement zum zerstörungsfreien Trennen von Nadeln der Nadeleinrichtung von der Platzierungseinrichtung aufweisen. Dies bietet den Vorteil, dass die Griffeinrichtung bzw. Platzierungseinrichtung leicht und anwenderfreundlich von der Nadeleinrichtung entfernt werden kann und dennoch eine sehr genaue Positionierung erfolgen kann.

Denkbar ist weiterhin eine Ausführungsform des hier vorgestellten Ansatzes, bei der die Nadeleinrichtung mittels zumindest eines Magnetkontakts mit einer Impulseinrichtung miteinander trennbar verbunden oder verbindbar ist, wobei die Impulseinrichtung, mit der Nadeleinrichtung zur Abgabe zumindest eines elektrischen Impulses an die Ohrmuschel und/oder an das Ohrläppchen koppelbar oder gekoppelt ist und/oder wobei die Nadeleinrichtung mittels zumindest einer Klammer oder Pinzette mit der Impulseinrichtung trennbar verbunden oder verbindbar ist. Die Impulseinrichtung - auch als impulsgebendes Gerät bezeichnet - kann dabei einen flexiblen Anschluss mit Magnetkontakten umfassen oder kann aus einem solchen Anschluss bestehen. Dieser flexible Anschluss kann oder muss bzw. wird dann mit den Magnetkontakten der Nadeleinrichtung verbunden werden. Eine solche magnetische Verbindung - hier auch lösbare Verbindung genannt - zwischen der Nadeleinrichtung und der Impulseinrichtung kann dafür sorgen, dass diese beiden Einrichtungen in der jeweils richtigen Position und Ausrichtung verbunden werden, indem sie beispielsweise einfach in die Nähe der jeweils anderen Einrichtung gebracht werden. Hierdurch kann eine komplizierte Handhabung der Verbindung durch den Anwender oder helfende Personen vermieden und stattdessen eine einfache Verbindung zwischen der Nadeleinrichtung und der Impulseinrichtung ermöglicht werden

Von Vorteil ist weiterhin eine Ausführungsform des hier vorgestellten Ansatezs, bei der die Nadeleinrichtung als zumindest eine Elektrodennadel und/oder als zumindest eine Mikronadel und/oder als Nadelelement mit zumindest zwei Nadeln ausgeformt ist, insbesondere wobei die zumindest eine Elektrodennadel und/oder die zumindest eine Mikronadel aus einem biokompatiblen Material, insbesondere aus einem biokompatiblen Metall, ausgeformt ist oder sind. Unter Verwendung einer spezifischen Form der Elektrodennadel und/oder der Mikronadel und/oder des Nadelelements - jeweils angepasst an die Form der Ohrmuschelregion bzw. des Ohrläppchens, in der die Nadel oder Nadeleinrichtung positioniert werden soll - mit einer abnehmbaren Griffeinrichtung wird beispielhaft eine korrekte Fixierung der Nadeln in der Ohrmuschel bzw. in oder an anderen Bereichen des Ohres durch den Anwender oder medizinisches Fachpersonal erreicht werden. Zugleich können die elektrischen Impulse sehr effizient und wirksam an das Ohr bzw. das Ohrläppchen abgegeben werden. Eine solche Variante bietet ebenfalls den Vorteil, dass die Nadeln auf unbedenkliche Weise für eine Therapie oder Behandlung eines Patienten in oder an dem Patienten angebracht werden können und an Stellen, an der die Nadeln in das Gewebe des Patienten eindringen keine Irritationen des Körpergewebes entstehen.

In einer anderen Ausführungsform kann die Nadeleinrichtung zumindest ein Klebeelement zum Befestigen der Nadeleinrichtung an der Ohrmuschel und/oder am Ohrläppchen aufweisen. Hierdurch lässt sich vor allem die Fixierung der Nadeleinrichtung an der Ohrmuschel und/oder am Ohrläppchen vereinfachen bzw. verbessern.

Auch schafft der hier vorgestellte Ansatz eine Nervenstimulationsvorrichtung zum elektrischen Stimulieren zumindest eines Nervs, zumindest einer Ohrmuschel und/oder zumindest eines Ohrläppchens. Die Nervenstimulationsvorrichtung weist die folgenden Merkmale auf:
- eine Nadeleinrichtung gemäß einer hier beschriebenen Variante, die ausgebildet ist, um an der Ohrmuschel und/oder am Ohrläppchen platziert zu werden oder platzierbar zu sein; und
- eine von der Nadeleinrichtung trennbare Impulseinrichtung zur Erzeugung von elektrischen Impulsen, wobei die Impulseinrichtung mit der Nadeleinrichtung zur Abgabe zumindest eines elektrischen Impulses an die Ohrmuschel und/oder an das Ohrläppchen koppelbar oder gekoppelt ist.

Die Nervenstimulationsvorrichtung kann zum Beispiel zur Verbesserung oder Behandlung von vielen von medizinischen Einsatzszenarios oder zur Erhöhung eines Nutzerkomforts eingesetzt werden, wie z.B. zur Linderung von Muskelverspannungen, zur Schmerzlinderung, bei peripheren arteriellen Gefäßerkrankungen, bei Entzündungen oder bei Vorhofflimmern.

Gemäß einer Ausführungsform kann die Impulseinrichtung ausgebildet sein, um elektrische Impulse in einem Stromstärkenbereich zwischen 0,05 Milliampere und 1,2 Milliampere abzugeben. Gemäß einer zusätzlichen Ausführungsform kann die Nervenstimulationsvorrichtung - auch Ohrnervenstimulationsgerät oder Stimulationseinheit genannt - mit einem elektrischen Strom von 0,4 Milliampere bis 1,2 Milliampere arbeiten. Gemäß einer weiteren Ausführungsform kann das Ohrnervenstimulationsgerät in einer Standard-Einstellung mit einem elektrischen Strom von 0,4 Milliampere arbeiten. Eine Abgabe elektrischer Impulse innerhalb dieses Stromstärkenbereichs ermöglicht die gewünschte Erregung der Axone in den feinen Nervenästen zwischen den beiden Nadeln, also zwischen Anode und Kathode.

Gemäß einer Ausführungsform kann die Impulseinrichtung ausgebildet sein, um elektrische Impulse in einem Frequenzbereich abzugeben, der einen Toleranzbereich um einen Frequenzwert von 1 Hertz repräsentiert. Diese Ausführungsform ist dadurch vorteilhaft, dass eine für die Wirkung der Nervenstimulationsvorrichtung besonders effektive Stimulationsfrequenz unkompliziert und schnell auf das Ohr bzw. das Ohrläppchen abgegeben werden kann.

Gemäß einer weiteren Ausführungsform kann die Impulseinrichtung zumindest ein Halteelement zum Fixieren der Impulseinrichtung am Ohr aufweisen. Das Halteelement kann hierbei auch als Montagehebel, lösbarer Montagehebel oder Montageklammer bezeichnet werden und vereinfacht bzw. verbessert das Befestigen der Impulseinrichtung am Ohr.

Gemäß einer Ausführungsform kann die Impulseinrichtung - auch Impulsgeber genannt - mit elektrischer Energie aufladbar sein. Mit anderen Worten kann die Impulseinrichtung nachladbar ausgeführt sein, insbesondere kontaktlos. Dies ist dadurch vorteilhaft, dass eine Stromversorgung der Impulseinrichtung anwenderfreundlich und unkompliziert möglich ist.

Gemäß einer weiteren Ausführungsform kann die Impulseinrichtung ausgebildet sein, um unter Verwendung einer Recheneinheit gesteuert oder steuerbar zu sein. Alternativ oder zusätzlich kann ein Steuerprogramm der Impulseinrichtung veränderbar sein. Als eine solche Recheneinheit und/oder als ein derartiges Steuerprogramm kann zum Beispiel ein Smartphone dienen. Lediglich beispielhaft kann die Impulseinrichtung - insbesondere drahtlos - mittels Code, welcher z.B. an und/oder über ein Smartphone beispielsweise kostenpflichtig übermittelt werden kann, für eine gewisse Betriebsdauer freigeschaltet werden. Diese Recheneinheit, die auch als Steuerung, Steuerprogramm oder Steuereinheit bezeichnet werden kann, kann beispielhaft entweder in das impulserzeugende Gerät eingebaut sein, das in der Nähe des Ohrs getragen wird, oder als eine weitere Möglichkeit softwarebasiert und/oder appbasiert in ein externes Gerät wie einen Computer oder ein Smartphone eingebaut sein, was noch praktischer sein kann. Ein derartiges externes Gerät kann eine softwarebasierte Steuerung durch den Anwender oder das medizinische Personal ermöglichen. Eine solche Steuerung kann zum Beispiel dazu verwendet werden, einen elektrischen Strom und/oder eine Frequenz einzustellen und zusätzlich oder alternativ eine Stimulationszeit und/oder eine Stimulationsfrequenz festzulegen. So könnte zum Beispiel über das Smartphone auch eine Behandlungsdauer eingestellt werden und das Gerät beispielsweise nachts um 24 Uhr abschalten, oder sich auf eine geringere Einzelpulsleistung oder eine niedrigere Pulsfrequenz oder auf längere Pulspausenintervalle einstellen. Im Falle einer softwarebasierten Recheneinheit kann dies beispielsweise mit verschiedenen Visualisierungen der Behandlungsmodi und/oder Zeiteinstellungen kombiniert werden. Diese softwarebasierte Steuerung kann auch verwendet werden, um Daten der Nervenstimulationsvorrichtung mit verschiedenen Biofeedback-Steuerungen anderer medizinischer und/oder nichtmedizinischer Messgeräte zu kombinieren.

Gemäß einer Ausführungsform kann die Impulseinrichtung ausgebildet sein, um eine Stimulation zumindest eines Ohrnervs am Außenohr und/oder eine Stimulation des Vagusnervs auszuführen. Die aurikuläre Elektrostimulation ist für die spezifische Stimulation des Vagusnervs vorteilhaft. Zu diesem Zweck können beispielsweise die Nadeleinrichtungen in Größe und Form so angepasst werden, dass sie in andere Bereiche der Ohrmuschel passen, sodass der Vagusnerv gezielt stimuliert werden kann.

Gemäß einer weiteren Ausführungsform kann die Impulseinrichtung ausgebildet sein, um hinter der Ohrmuschel befestigt zu werden. Lediglich beispielhaft kann die Impulseinrichtung durch eine Miniaturisierung derselben kaum wahrnehmbar hinter oder in der Ohrmuschel untergebracht werden, ohne Tragediskomfort zu verursachen. Somit ist eine bequeme Platzierung bzw. ein sicherer Halt der Impulseinrichtung hinter oder in der Ohrmuschel möglich. Im Falle einer miniaturisierten Ausführung der Impulseinrichtung spürt der Anwender bestenfalls nicht einmal, dass er die Impulseinrichtung am Ohr trägt.

Günstig ist weiterhin eine Ausführungsform des hier vorgestellten Ansatzes, bei der ein Lautsprecher vorgesehen ist, insbesondere wobei der Lautsprecher an oder in der Nadeleinrichtung und/oder der Impulseinrichtung angeordnet ist. Auf diese Weise kann die Nervenstimulationsvorrichtung mit einem Ohrhörer bzw. einem Hörgerät kombiniert werden, sodass ein Mehrfach-Nutzen der Nervenstimulationsvorrichtung auch für andere Zwecke erreicht werden kann. In einer weiteren Ausführungsform des hier vorgestellten Ansatzes ist die Impulseinrichtung kombiniert mit einem Lautsprecher und/oder ergänzt durch "in-ear-Kopfhörer", wobei durch eine Anpassung der Stimulator-Signale an z.B. den Rhythmus der Musik eine höhere Compliance in der Anwendung, ein höherer Tragekomfort oder eine verbesserte Wirkung erreicht wird.

Die vorstehend genannten Vorteile lassen sich auch in einer Ausführungsform des hier vorgestellten Ansatzes als Verfahren zur Verwendung einer hier beschriebenen Variante einer Nadeleinrichtung realisieren, wobei das Verfahren den Schritt des Platzierens der Nadeleinrichtung an der Ohrmuschel und/oder am Ohrläppchen eines Nutzers unter Verwendung der Griffeinrichtung aufweist.

Besonders günstig ist hierbei eine Ausführungsform des vorgestellten Ansatzes, bei dem ein Schritt des Trennens der Griffeinrichtung (400) an einer Sollbruchstelle (600) vorgesehen ist. Auf diese Weise kann eine effiziente und praktikable Platzierung der Nadeleinrichtung an der Ohrmuschel und/oder am Ohrläppchen eines Nutzers vorgenommen werden, wobei die Griffeinrichtung nachfolgend aus Gründen des besseren Komforts des Nutzers oder der Nutzerin der Nadeleinrichtung wieder entfernt werden kann.

Ausführungsbeispiele des präsentierten Ansatzes sind in den Zeichnungen rein schematisch dargestellt und werden nachfolgend näher beschrieben. Es zeigt
Figur 1 eine schematische Darstellung eines Ausführungsbeispiels einer Nervenstimulationsvorrichtung mit einer Nadeleinrichtung gemäß einem Ausführungsbeispiel;
Figur 2 eine schematische Darstellung eines Ausführungsbeispiels einer Nervenstimulationsvorrichtung;
Figur 3 eine schematische Darstellung eines Ausführungsbeispiels einer Nervenstimulationsvorrichtung;
Figur 4 eine schematische Darstellung eines Ausführungsbeispiels einer Nervenstimulationsvorrichtung mit einer Nadeleinrichtung gemäß einem Ausführungsbeispiel und einer Griffeinrichtung;
Figur 5 eine schematische Darstellung eines Ausführungsbeispiels einer Nervenstimulationsvorrichtung mit einer Nadeleinrichtung gemäß einem Ausführungsbeispiel;
Figur 6a eine schematische Darstellung einer Nadeleinrichtung gemäß einem Ausführungsbeispiel und einer Griffeinrichtung zur Verwendung in einem Ausführungsbeispiel einer Nervenstimulationsvorrichtung;
Figur 6b eine perspektivische Darstellung einer Nadeleinrichtung und einer Griffeinrichtung zur Verwendung in einem Ausführungsbeispiel einer Nervenstimulationsvorrichtung;
Figur 6c eine schematische Darstellung ein einer Nadeleinrichtung gemäß einem Ausführungsbeispiel und einer Griffeinrichtung zur Verwendung in einem Ausführungsbeispiel einer Nervenstimulationsvorrichtung;
Figur 6d vier schematische Teildarstellungen jeweils einer Nadeleinrichtung gemäß einem Ausführungsbeispiel und einer Griffeinrichtung zur Verwendung in einem Ausführungsbeispiel einer Nervenstimulationsvorrichtung;
Figur 6e verschiedene perspektivische Darstellungen einer Platzierungseinheit zur Platzierung der Nadeleinrichtung an einem Ohr;
Figur 7a in einem oberen Teilbereich eine schematische Teildarstellung einer Nadeleinrichtung gemäß einem Ausführungsbeispiel und einer Griffeinrichtung zur Verwendung in einem Ausführungsbeispiel einer Nervenstimulationsvorrichtung und in einem mittleren und einem unteren Teilbereich jeweils eine perspektivische Teildarstellung jeweils einer Nadeleinrichtung gemäß einem Ausführungsbeispiel und einer Griffeinrichtung zur Verwendung in einem Ausführungsbeispiel einer Nervenstimulationsvorrichtung;
Figur 7b zwei Darstellungen von möglichen Ausführungsbeispielen für eine Geometrie der Nadeln;
Figur 8 eine perspektivische Darstellung eines Ausführungsbeispiels einer Nervenstimulationsvorrichtung;
Figur 9 drei schematische Teildarstellungen jeweils eines Ausführungsbeispiels einer Nervenstimulationsvorrichtung mit einer Impulseinrichtung;
Figur 10 eine schematische Darstellung eines Ausführungsbeispiels einer Nervenstimulationsvorrichtung;
Figur 11a bis 11g je eine Ansicht eines Ausführungsbeispiels einer Nervenstimulationsvorrichtung;
Figuren 12a bis 12g je eine Ansicht eines Ausführungsbeispiels einer Impulseinrichtung;
Figur 13a bis 13g je eine Ansicht eines Ausführungsbeispiels einer Impulseinrichtung; und
Figur 14a bis 14g je eine perspektivische Darstellung eines Ausführungsbeispiels der Nervenstimulationsvorrichtung sowie der Anordnung der Nervenstimulationsvorrichtung an einem Kopf eines Nutzers.

Gleiche oder ähnliche Bezugszeichen werden in der nachfolgenden Beschreibung für gleiche oder ähnliche Elemente verwendet, wobei auf eine wiederholte Erläuterung der Funktion dieser Elemente aus Gründen der Übersichtlichkeit verzichtet wird.

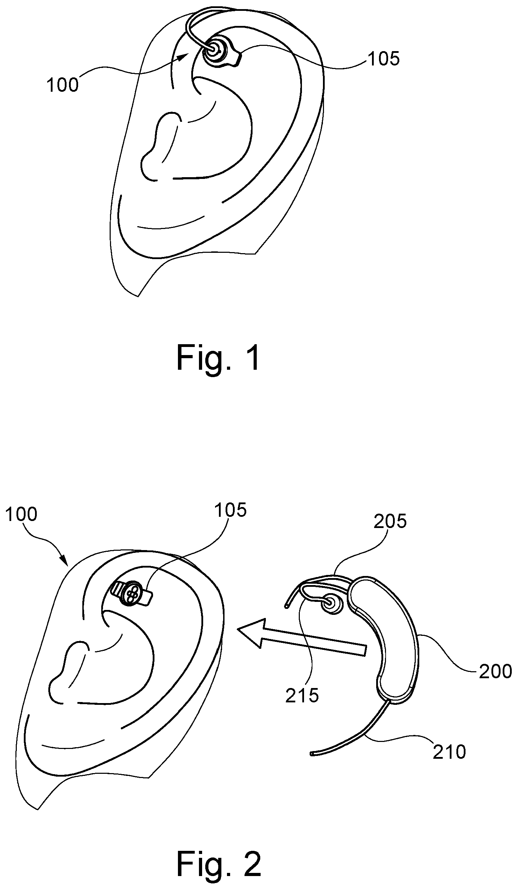

Figur 1 zeigt eine schematische Darstellung eines Ausführungsbeispiels einer Nervenstimulationsvorrichtung 100 mit einer Nadeleinrichtung 105. Unter Verwendung der Nervenstimulationsvorrichtung 100 wird zumindest ein Nerv an einer Ohrmuschel elektrisch stimuliert, an welcher die Nadeleinrichtung 105 platziert ist.

Anders ausgedrückt zeigt Figur 1 eine Abbildung der Positionierung der Nadeleinheit im Ohr nach Fixierung im Ohr.

Verschiedene Ausführungsbeispiele des vorliegenden Ansatzes können Nadeleinrichtungen unterschiedlicher Größe und/oder Form umfassen. Solche Nadeleinrichtungen können in ihrer Form an bestimmte Teile der Ohrmuschel wie zum Beispiel die *Fossa triangularis* bzw. die Ohrläppchen angepasst sein. Mit anderen Worten kann die Form der Nadeleinrichtung so angepasst sein, dass sie in der *Fossa triangularis* platziert werden kann.

Die Nadeleinrichtungen können auch in Größe und Form so angepasst werden oder sein, dass sie in andere Bereiche der Ohrmuschel passen, um zum Beispiel den *Nervus Vagus,* also den Vagusnerv, gezielt zu stimulieren.

Figur 2 zeigt eine schematische Darstellung eines Ausführungsbeispiels einer Nervenstimulationsvorrichtung 100. Unter Verwendung der Nervenstimulationsvorrichtung 100 wird zumindest ein Nerv an einer Ohrmuschel elektrisch stimuliert, an welcher die Nadeleinrichtung 105 platziert ist.

Eine Impulseinrichtung 200 ist hier mit der Nadeleinrichtung 105 gekoppelt und erzeugt elektrische Impulse an die Ohrmuschel. Gemäß einem Ausführungsbeispiel können diese Impulse in einem Stromstärkenbereich zwischen 0,05 Milliampere und 1,2 Milliampere abgegeben werden. Gemäß einem zusätzlichen Ausführungsbeispiel können diese Impulse in einem Frequenzbereich abgegeben werden, der einen Toleranzbereich um einen beispielhaften Frequenzwert von 1 Hertz repräsentieren kann. Gemäß einem Ausführungsbeispiel ist die Impulseinrichtung 200 mit elektrischer Energie aufladbar. Lediglich beispielhaft kann die Impulseinrichtung 200 unter Verwendung einer in dieser Figur 2 nicht dargestellten Recheneinheit gesteuert werden. Alternativ oder zusätzlich dazu kann ein in dieser Figur 2 nicht dargestelltes Steuerprogramm der Impulseinrichtung 200 veränderbar sein. Gemäß einem weiteren Ausführungsbeispiel kann die Impulseinrichtung 200 zumindest einen Ohrnerv am Außenohr und/oder den Vagusnerv stimulieren. Die Impulseinrichtung 200 umfasst ein Halteelement 205, unter dessen Verwendung sich die Impulseinrichtung 200 am Ohr fixieren lässt. Darüber hinaus umfasst die Impulseinrichtung 200 eine Anschlussleitung 210 für einen Anschluss der Impulseinrichtung 200 zum Beispiel an die vorgenannte Recheneinheit. Die Impulseinrichtung 200 umfasst zudem eine Verbindungsleitung 215 zum Verbinden der Impulseinrichtung 200 mit der Nadeleinrichtung 105. Die Verbindungsleitung 215 kann alternativ als Anschluss an impulsgebendes Gerät bezeichnet werden.

Anders ausgedrückt zeigt Figur 2 eine Visualisierung des impulsgebenden Geräts und seiner Befestigung am Ohr.

Eine Miniaturisierung der Nervenstimulationsvorrichtung kann es gewährleisten oder ermöglichen, dass die Nervenstimulationsvorrichtung, die auch als Gerät zur Elektrostimulation bezeichnet werden kann, an einem Ohr oder in der Nähe eines Ohrs getragen werden kann, zum Beispiel indem die Impulseinrichtung am oder hinter dem Ohr getragen wird und die zumindest eine Nadel in der Ohrmuschel und/oder an dem Ohrläppchen an einer gewünschten Stelle positioniert werden. Die Impulseinrichtung, die auch Impulsgeber genannt werden kann, kann beispielsweise mit einem kleinen Griff ausgestattet sein, der eine Fixierung des Geräts während des Gebrauchs gewährleisten oder verbessern kann.

Gemäß einem Ausführungsbeispiel kann die Impulseinrichtung - auch Impulsgeber oder lösbarer Impulsgeber genannt - in einer Einheit mit der beispielsweise doppelten Elektrodeneinheit sein. Ratsam können hier Miniaturisierung, kleine Batterie, wiederaufladbar, evtl. auch kontaktlos aufladbar sein. Bei diesem Ausführungsbeispiel kann es möglich sein, die Elektrode von hinten am Ohr zu befestigen. Bei einem solchen Ausführungsbeispiel kann es auch möglich sein, lediglich beispielhaft eine Elektrode hinter dem Ohr und eine weitere in der Ohrmuschel zu platzieren.

Gemäß einem Ausführungsbeispiel können Mikroelektroden verwendet werden, die die oberste Hautschutzschicht durchdringen. Bei diesem Ausführungsbeispiel kann ein großer Teil der Widerstandsschwankungen beseitigt werden, welche z. B. durch unterschiedliche Oberflächeneigenschaften wie etwa Cremes oder Fett verursacht werden können. Hier lässt sich beispielsweise auch eine Elektrode außen und eine zweite Elektrode hinter dem Ohr anbringen. Die beiden Elektroden lassen sich (dann) zum Beispiel mit einer elastischen oder federnden Klammer oder Halterung verbinden.

Gemäß einem weiteren Ausführungsbeispiel kann es auch möglich sein, mehrere Elektrodenpaare gleichzeitig zu verwenden, um beispielsweise eine bessere Abdeckung der Nervenäste erreichen zu können. Hier kann auch eine ungerade Anzahl von Elektroden verwandt werden. Ein Leiter könnte zum Beispiel zu zwei Elektroden und ein weiterer Leiter zu einer Elektrode führen.

Gemäß einem weiteren Ausführungsbeispiel kann die Elektrode so konzipiert sein, dass sie eine gezielte Innervation bestimmter Nervenstrukturen, die lediglich beispielhaft ausschließlich in der Fossa triangularis zu finden sind, auf einfach(st)e Weise ermöglichen kann. Beispielsweise in Kombination mit einem mikroprozessorgesteuerten Stimulator kann die Elektrode einen gewünschten therapeutischen Erfolg bei der Behandlung verschiedenster Erkrankungen erzielen. Beispielshalber durch eine spezielle Konstruktion, bei der ein Abstand zur Innenspirale und eine integrierte Doppelnadel in einem Abstand von zwei Millimetern konstruiert sein können, kann eine zeitaufwendige Nervensuche entfallen, was aber die Funktion einer Nervenstimulation des richtigen Nervengeflechts garantieren kann. Dieser spezielle Plexus ist ein rein afferentes (sensorisches) Nervengeflecht, das über C2 und C3 in den Nucleus tractus solitarii mündet, wo zum Beispiel eine Modulation von Neuronen und Synapsen möglich sein kann. Ein spezielles Design der Elektrode kann zum einen garantieren, dass die richtige Nervenstruktur aktiviert wird und zum anderen, dass keine Nerven stimuliert werden, bei denen unerwünschte Nebenwirkungen auftreten können. So kann beispielsweise eine reine Nervus-Vagus-Stimulation zu einer Verlangsamung der Herzfrequenz führen.

Darüber hinaus kann eine Platzierung der Elektrode in der Fossa triangularis sehr gut für eine Langzeittherapie geeignet sein. Wenn die Elektrode in einer Vertiefung der Ohrmuschel in einem weitgehend schmerzfreien Bereich platziert ist, ist ein Aufliegen im Schlaf möglich. Die Elektrode kann beispielhaft so klein sein, dass sie auch in die kleinste Fossa triangularis mit steil ansteigenden Flanken passt. Eine Vorteilhaftigkeit des Designs der Elektrode zeigt sich in der Gesamtkonzeption der Elektrodenform, die einen Abstand zur Innenseite der Helix garantieren kann, so dass eine bestmögliche Platzierung der Elektrode im oder am Ohr gewährleistet sein kann.

Als besonders hilfreich beim Setzen der Elektrode ist ein als Anker ausgebildeter Fortsatz. Dieser ist als Distanzhalter zum Ohrbogen bei der Fossa triangularis unter der Helix günstig verwendbar, um eine exakte Positionierung der Elektrode zu gewährleisten. Andererseits ist dieser Fortsatz auch als Fixierungshilfe beim Setzen der Elektrode äußerst hilfreich.

Hier handelt es sich um eine Hybrid-Elektrode, bei der oben benannte Ankerfortsatz als Oberflächen-Elektrode ausgeführt ist. Die zweite Elektrode (Anode-Kathode) ist als Nadel ausgeführt, die jedoch, mit Ausnahme der Nadelspitze, isoliert sein sollte.

Der Vorteil ist, dass eine Nadel weniger Schmerz verursacht.

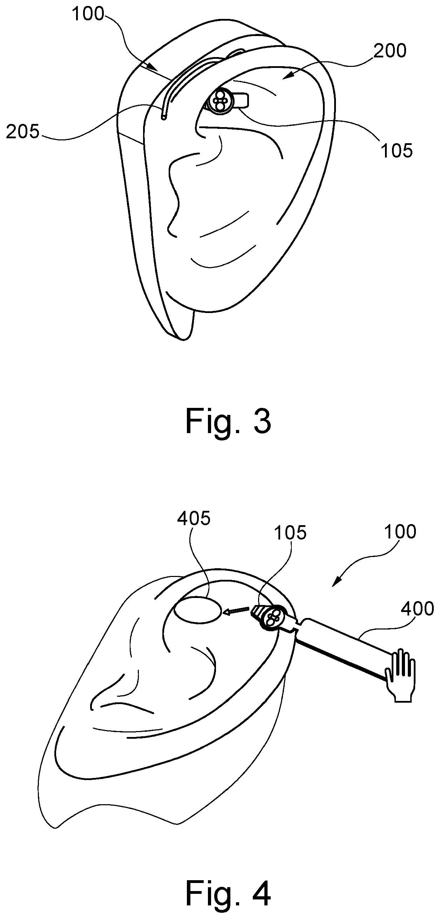

Figur 3 zeigt eine schematische Darstellung eines Ausführungsbeispiels einer Nervenstimulationsvorrichtung 100. Unter Verwendung der Nervenstimulationsvorrichtung 100 wird zumindest ein Nerv an einer Ohrmuschel elektrisch stimuliert, an welcher die Nadeleinrichtung 105 platziert ist. Die in Figur 2 näher beschriebene Impulseinrichtung 200 oder eine ähnliche Impulseinrichtung ist unter Verwendung eines Halteelements 205 hinter der Ohrmuschel befestigt.

Anders ausgedrückt zeigt Figur 3 eine Visualisierung des impulsgebenden Geräts und seiner Befestigung am Ohr.

Figur 4 zeigt eine schematische Darstellung eines Ausführungsbeispiels einer Nervenstimulationsvorrichtung 100 mit einer Nadeleinrichtung 105 und einer Griffeinrichtung 400. Die Nadeleinrichtung 105 kann beispielhaft unter Verwendung der Griffeinrichtung 400 und eines Klebeelements 405 an der Ohrmuschel befestigt werden. Dementsprechend kann die Griffeinrichtung 400 als eine andere Möglichkeit auch Handgriff für die Manipulation genannt werden.

Anders ausgedrückt zeigt Figur 4 eine Visualisierung der Manipulation der Nadeleinheit mit dem Applikator in die gewünschte Position, z. B. die Fossa triangularis.

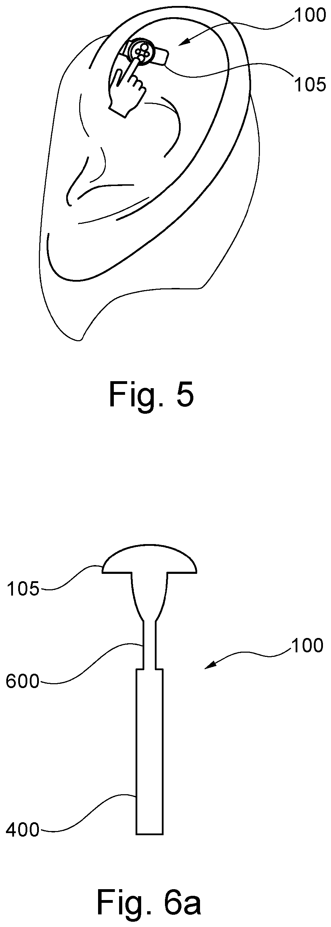

Figur 5 zeigt eine schematische Darstellung eines Ausführungsbeispiels einer Nervenstimulationsvorrichtung 100 mit einer Nadeleinrichtung 105. Die Nadeleinrichtung 105 lässt sich durch einen Anwender selbst oder durch medizinisches Personal in einer gewünschten Position beispielsweise in der Ohrmuschel fixieren. Die in Figur 4 illustrierte Griffeinrichtung lässt sich (anschließend) entfernen.

Anders ausgedrückt zeigt Figur 5 eine Visualisierung der Fixierung der Nadeleinheit in der gewünschten Position, gefolgt von der Entfernung des Applikators.

Figur 6a zeigt eine schematische Darstellung einer Nadeleinrichtung 105 und einer Griffeinrichtung 400 zur Verwendung in einem Ausführungsbeispiel einer Nervenstimulationsvorrichtung 100. Die Griffeinrichtung 400 kann alternativ als abnehmbarer Griff, um die Nadeleinrichtung 105 in eine gewünschte Position zu bringen, bezeichnet werden.

Gemäß einem Ausführungsbeispiel kann die Nadeleinrichtung 105 als Elektrodennadel oder als Mikronadel ausgeformt sein. Gemäß einem zusätzlichen Ausführungsbeispiel kann die Elektrodennadel oder die Mikronadel aus einem biokompatiblen Material, insbesondere aus einem biokompatiblen Metall, ausgeformt sein.

Überdies weist die Nadeleinrichtung 105 eine insbesondere vorbestimmte Sollbruchstelle 600 zum Trennen der Griffeinrichtung 400 von der Nadeleinrichtung 105 auf.

Gemäß einem Ausführungsbeispiel kann eine Form der Nadeleinrichtung 105 an einen Anwendungsbereich der Ohrmuschel, z. B. die Fossa triangularis, angepasst oder anpassbar sein.

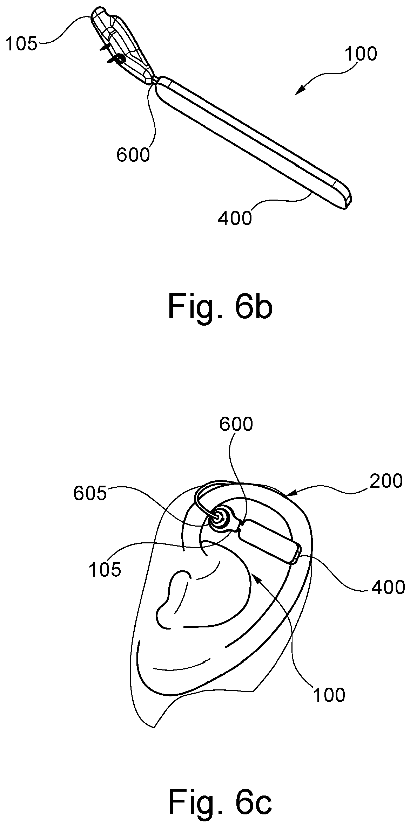

Figur 6b zeigt eine perspektivische Darstellung einer Nadeleinrichtung 105 und einer Griffeinrichtung 400 zur Verwendung in einem Ausführungsbeispiel einer Nervenstimulationsvorrichtung 100. Gemäß einem Ausführungsbeispiel kann die Nadeleinrichtung 105 als Nadelelement mit zwei Nadeln ausgeformt sein. Gemäß einem zusätzlichen Ausführungsbeispiel können die Elektrodennadeln auf 2,0 Millimeter voneinander entfernt sein und/oder eine Länge von 2,0 Millimetern aufweisen. Zum Beispiel eine doppelte Nadel, beispielhalber 2 Millimeter zwischen Anode und Kathode, und eine Abstandsverlängerung in Form eines Ankers können eine einfache, aber vor allem korrekte Platzierung der Elektrode gewährleisten.

Gemäß einem weiteren Ausführungsbeispiel können die Elektroden ein oder mehrere biokompatible(s) Metall(e) wie zum Beispiel Titan aufweisen.

Gemäß einem Ausführungsbeispiel kann die Elektrode - auch als Elektrodeneinheit oder selbstfixierbare Elektrodeneinheit bezeichnet - in einem gewünschten Bereich durch einen geeigneten, gegebenenfalls trennbaren, Montageabstandhalter oder eine Montageklammer fixiert werden.

Gemäß einem weiteren Ausführungsbeispiel könnte das Nadelelement mit zumindest zwei Nadeln - auch als Doppelnadelelektrode bezeichnet - beispielsweise durch zwei Mikronadeln ersetzt werden, von denen sich lediglich beispielhaft eine Mikronadel im Ohr und die andere Mikronadel hinter dem Ohr befinden kann. Dies würde es den Stimulationssignalen ermöglichen, trotz etwaiger Unterschiede in der Oberflächenbeschaffenheit der Haut und trotz der Fettschicht der Haut in die Haut einzudringen, um einen Stromübertragungswiderstand nach Möglichkeit ähnlich zu halten. Auch ein Infektionsrisiko aufgrund einer gewissen Hautbarriere kann durch eine Verwendung der kurzen Mikronadeln im Vergleich zu einer bekannten Lösung, 2 Millimeter lange Nadeln zu verwenden und den Strom (dann) durch die nervenbedeckte Knorpelzone zu leiten, minimiert werden. Technisch ließe sich dies z. B. durch Aufkleben oder Anbringen eines Zugstabs erreichen, während die Elektronik in miniaturisierter Form als eine Möglichkeit hinter dem Ohr platziert werden könnte.

Anatomische Darstellungen der Nervenstruktur in der Fossa triangularis zeigen, dass an dieser Stelle ein so dichtes Nervengeflecht vorhanden ist, dass ein über die Doppelnadel abgegebener Stromimpuls im Optimalfall immer oder zumindest sehr häufig eine gewünschte Erregung der Axone in den feinen Nervenästen zwischen den beiden Nadeln, also Anode und Kathode, mit sehr geringen Stromstärken ermöglichen kann.

Durch eine derartige Konstruktion, welche auch als Zwei-Nadel-Konstruktion bezeichnet werden kann, spürt der Patient während einer Therapie im Idealfall keine Stromimpulse, da die empfindlichen punktuellen Nervenenden auf der Hautoberfläche weit genug entfernt sind. Damit kann es möglich sein, medizinische Doppelblindstudien durchzuführen, die von Zulassungsbehörden für Geräte zur minimalinvasiven Stimulationstherapie gefordert werden können.

Das folgende Beispiel kann dazu dienen, dem Fachmann eine möglichst vollständige und anschauliche Offenlegung und Beschreibung einer Herstellung eines Ausführungsbeispiels einer Nervenstimulationsvorrichtung zu geben und dazu, eine Wirkungsweise der Nervenstimulationsvorrichtung zu veranschaulichen, welche auch als Gerät zur Abgabe von elektrischen Impulsen bezeichnet werden kann.

Überdies weist die Nadeleinrichtung 105 eine Sollbruchstelle 600 zum Trennen der Griffeinrichtung 400 von der Nadeleinrichtung 105 auf.

Anders ausgedrückt zeigt Figur 6b eine Abbildung der Kombination aus Nadeleinheit, abnehmbarem Griff zur Handhabung und der Sollbruchstelle dazwischen.

Figur 6c zeigt eine schematische Darstellung einer Nadeleinrichtung 105 und einer Griffeinrichtung 400 zur Verwendung in einem Ausführungsbeispiel einer Nervenstimulationsvorrichtung 100. Die Nadeleinrichtung 105 und die hinter der Ohrmuschel befindliche Impulseinrichtung 200 sind über einen Magnetkontakt 605 miteinander verbunden.

Überdies weist die Nadeleinrichtung 105 eine Sollbruchstelle 600 zum Trennen der Griffeinrichtung 400 von der Nadeleinrichtung 105 auf.

Anders ausgedrückt zeigt Figur 6c eine Abbildung der Positionierung der Nadeleinheit im Ohr mit dem Manipulationsgriff. Die Griffeinrichtung - alternativ Führungsstab genannt - mit seiner Sollbruchstelle kann eine einfache und sterile Platzierung der Elektrode im oder am Ohr garantieren. Durch ein Abbrechen des Führungsstabes ist eine wiederholte, möglicherweise unsterile Anwendung kaum möglich.

In anderen Worten zeigt Figur 6c eine Handhabung einer Fixierung der Nadeln. Eine technische und/oder medizinische Herausforderung, die es zu lösen gilt, ist die korrekte Fixierung der Nadeln in der Ohrmuschel bzw. in oder an anderen Bereichen des Ohres durch den Anwender oder medizinisches Personal. Gemäß einem Ausführungsbeispiel der Nervenstimulationsvorrichtung des vorliegenden Ansatzes kann dies durch eine Verwendung einer Nadeleinrichtung mit einer spezifischen Form - angepasst an eine Form der Ohrmuschelregion bzw. des Ohrläppchens, in der oder dem die Nadeleinrichtung beispielhaft positioniert werden soll - mit einer beispielsweise abnehmbaren Griffeinrichtung - auch Griff genannt - erreicht werden. Durch eine Manipulation der Nadeleinrichtung mit dem Griff können die Nadeln an einer gewünschten Stelle zum Beispiel in der Ohrmuschel positioniert werden. Ist dies geschehen, können die Nadeln in die Haut gedrückt und so fixiert werden. Die Form der Nadeleinrichtung kann an bestimmte anatomische Bereiche der Ohrmuschel angepasst werden, um die Fixierung in einem bestimmten Bereich zu erleichtern, zum Beispiel in der Fossa triangularis. Für eine Positionierung und Befestigung am Ohrläppchen kann dagegen eine gegebenenfalls daran angepasste Form und Dimension erstrebenswert sein. Gemäß einem Ausführungsbeispiel kann die Nadeleinrichtung mit einem in der vorigen Figur 4 dargestellten und dort näher beschriebenen Klebeelement, das auch als Klebeverbindung bezeichnet werden kann, ausgestattet sein, um die Fixierung zu verbessern. Nach der Fixierung kann eine Verbindung zwischen der Nadeleinrichtung und der Griffeinrichtung - auch als Manipulationsgriff bezeichnet - an einer Sollbruchstelle unterbrochen werden. Für eine Verbindung mit der Impulseinrichtung - alternativ impulsgebende Vorrichtung genannt - kann die Nadeleinrichtung beispielhaft einen oder mehrere Magnetkontakt(e) umfassen.

Die Nadeleinrichtungen können Griffeinrichtungen - auch abnehmbarer Griff oder Applikator genannt - unterschiedlicher Größe und Form umfassen, die so angebracht sein können, dass sie leicht entfernt werden können, wenn die Nadeleinrichtung fixiert wurde, zum Beispiel durch eine Sollbruchstelle. Darüber hinaus können die Nadeleinrichtungen beispielhaft mit einem in der vorigen Figur 4 dargestellten und dort näher beschriebenen Klebeelement - alternativ Klebeverbindung genannt - versehen sein, um die Fixierung zu erleichtern. Ausführungsbeispiele des vorliegenden Ansatzes können auch miniaturisierte impulserzeugende Vorrichtungen umfassen, die am Ohr getragen werden können. Darüber hinaus können Nadeleinrichtung und impulserzeugende Vorrichtung beispielsweise magnetische Kontakte umfassen, durch die sie verbunden sein können.

Kurz gesagt können die Ausführungsbeispiele eine Kombination aus einem miniaturisierten impulserzeugenden Gerät und einer Nadeleinrichtung, die über Magnetkontakte verbunden sein kann, umfassen. Die Nadeleinrichtung kann beispielhaft eine an den aurikulären Anwendungsbereich angepasste Form aufweisen und/oder einen abnehmbaren Griff zur Fixierung im Ohr enthalten. Die Steuerung der Nervenstimulationsvorrichtung kann zum Beispiel direkt durch das impulserzeugende Gerät - auch impulserzeugende Einheit genannt - oder softwarebasiert über ein externes Gerät wie einen Computer oder ein Smartphone erfolgen. Ausführungsbeispiele gemäß des vorliegenden Ansatzes können zur Verbesserung oder Behandlung einer Vielzahl medizinischer und nicht-medizinischer Zustände wie der Linderung von Muskelverspannungen, der Linderung von Schmerzen, peripheren arteriellen Gefäßerkrankungen, entzündlichen Zuständen oder Vorhofflimmern angewendet werden.

Ausführungsbeispiele des vorliegenden Ansatzes können Nadeleinrichtungen unterschiedlicher Größe und Form umfassen, welche in ihrer Form zum Beispiel an bestimmte Teile der Ohrmuschel angepasst werden können, z. B. die *Fossa triangularis, den* Helixschwanz, die Scapha, den Antitragus, die Cymba conchae, die Antihelix oder das Ohrmuschelläppchen. Diese Nadeleinrichtungen können beispielsweise eine Griffeinrichtung - auch abnehmbarer Griff oder Applikator genannt - unterschiedlicher Größe und Form umfassen. Diese Griffeinrichtung kann so angebracht sein, dass sie leicht entfernt werden kann, wenn die Nadeleinrichtung fixiert wurde, z. B. indem sie eine Sollbruchstelle aufweist. Darüber hinaus können die Nadeleinrichtungen ein in der vorigen Figur 4 dargestelltes und dort näher beschriebenes Klebeelement umfassen, um eine Fixierung zu erleichtern. Ausführungsbeispiele des vorliegenden Ansatzes können auch beispielsweise miniaturisierte impulserzeugende Vorrichtungen, die am Ohr getragen werden, umfassen. Außerdem können Nadeleinrichtung und/oder impulsgebende Vorrichtung magnetische Kontakte aufweisen, mit denen sie verbunden werden können.

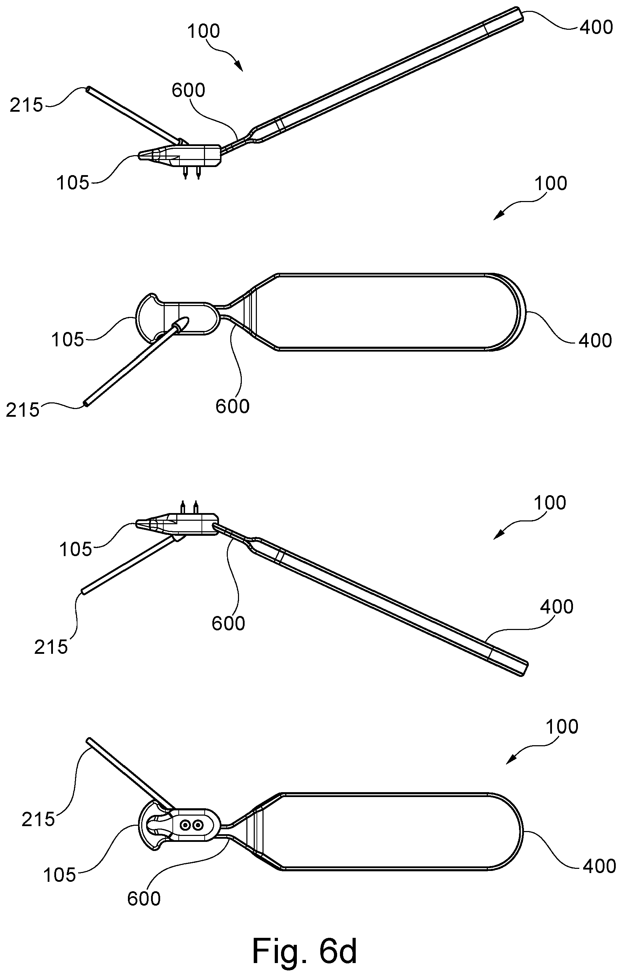

Figur 6d zeigt vier schematische Teildarstellungen jeweils ei einer Nadeleinrichtung 105 und einer Griffeinrichtung 400 zur Verwendung in einem Ausführungsbeispiel einer Nervenstimulationsvorrichtung 100. Davon ausgehend, dass die Nervenstimulationsvorrichtung 100 in der von oben her ersten Teildarstellung von links dargestellt ist, ist die Nervenstimulationsvorrichtung 100 in der von oben her zweiten Teildarstellung von oben dargestellt, also im Vergleich zur ersten Teildarstellung um 90 Grad gedreht. In der von oben her dritten Teildarstellung ist die Nervenstimulationsvorrichtung 100 von rechts dargestellt, also im Vergleich zur zweiten Teildarstellung um 90 Grad gedreht. In der von oben her vierten Teildarstellung ist die Nervenstimulationsvorrichtung 100 von unten dargestellt, also im Vergleich zur dritten Teildarstellung um 90 Grad gedreht.

Gemäß einem Ausführungsbeispiel kann die Nadeleinrichtung 105 als Nadelelement mit zwei Nadeln ausgeformt sein. Überdies weist die Nadeleinrichtung 105 eine Sollbruchstelle 600 zum Trennen der Griffeinrichtung 400 von der Nadeleinrichtung 105 auf. Die Nadeleinrichtung 105 umfasst zudem eine Verbindungsleitung 215 zum Verbinden der Nadeleinrichtung 105 mit der in dieser Figur 6d nicht illustrierten Impulseinrichtung.

Anders ausgedrückt zeigen die Teildarstellungen der Figur 6d Abbildungen der Kombination aus Nadeleinheit und abnehmbarem Griff für die Manipulation und der Sollbruchstelle zwischen ihnen, und zwar von beiden Seiten sowie von oben und von unten.

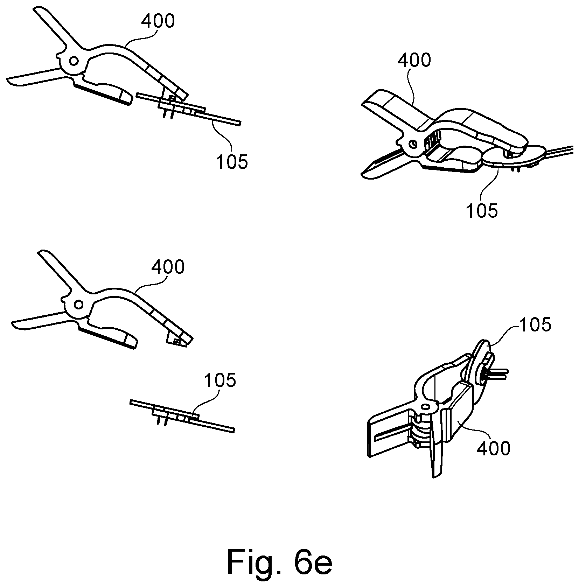

Figur 6e zeigt verschiedene perspektivische Darstellungen einer Platzierungseinrichtung 400 (bzw. synonym Platzierungseinheit) zur Platzierung der Nadeleinrichtung 105 an einem Ohr. Die Platzierungseinrichtung 400 kann dabei der Griffeinheit 400 entsprechen oder alternativ zu der Griffeinheit 400 verwendet werden und ist aus diesem Grund mit dem gleichen Bezugszeichen 40 versehen. Die Platzierungseinrichtung 400 ist jedoch entgegen der Griffeinheit 400 nicht direkt mit der Nadeleinrichtung 105 verbunden und wird nicht durch ein Abbrechen der Griffeinheit 400 bzw. Platzierungseinheit von der Nadeleinheit 105 getrennt, sondern kann durch einen Magnet-, Schnapp- und/oder Klickverschluss bzw. Bajonettverschluss von der Nadeleinheit 105 getrennt werden. Die Verwendung der Platzierungseinheit 400 ist hierbei vorteilhaft, wenn eine sehr präzise und genaue Platzierung der Nadeleinheit 105 am oder im Ohr des Patienten oder allgemein des Nutzers erfolgen soll, da die Platziereinheit in der Art einer Pinzette oder Klammer eine sehr genaue Positionierung der Nadeleinheit 105 an einer gewünschten Position im oder am Ohr ermöglicht, die auch bei einem Lösen und einer Entfernung der Platziereinheit 400 stabile gehalten werden kann.

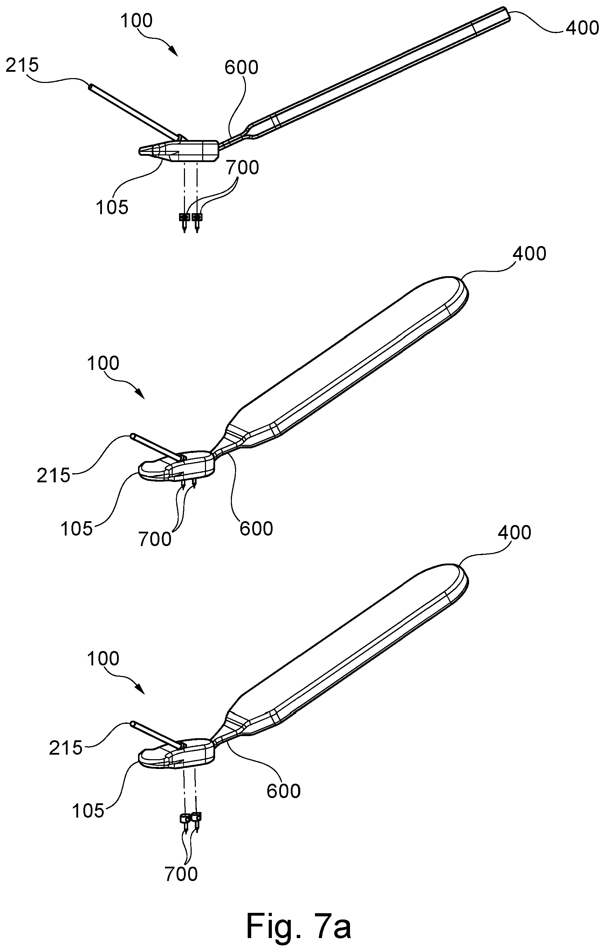

Figur 7a zeigt in einem oberen Teilbereich eine schematische Teildarstellung einer Nadeleinrichtung 105 und einer Griffeinrichtung 400 zur Verwendung in einem Ausführungsbeispiel einer Nervenstimulationsvorrichtung 100 und in einem mittleren und einem unteren Teilbereich jeweils eine perspektivische Teildarstellung jeweils einer Nadeleinrichtung 105 und einer Griffeinrichtung 400 zur Verwendung in einem Ausführungsbeispiel einer Nervenstimulationsvorrichtung 100.

Gemäß Ausführungsbeispielen kann die Nadeleinrichtung 105 in allen drei Teildarstellungen jeweils als Nadelelement mit zwei Nadeln 700 ausgeformt sein. Überdies weist die Nadeleinrichtung 105 in allen drei Teildarstellungen jeweils eine Sollbruchstelle 600 zum Trennen der Griffeinrichtung 400 von der Nadeleinrichtung 105 auf. Die Nadeleinrichtung 105 umfasst zudem eine Verbindungsleitung 215 zum Verbinden der Nadeleinrichtung 105 mit der in dieser Figur 7a nicht illustrierten Impulseinrichtung.

Anders ausgedrückt zeigen die Teildarstellungen der Figur 7a Abbildungen der Kombination aus Nadeleinheit, abnehmbarem Griff für die Handhabung und der Sollbruchstelle zwischen ihnen. Diese Abbildungen der Figur 7a veranschaulichen die Verbindung der Nadeln 700. In der oberen und in der unteren Teildarstellung sind die Nadeln 700 jeweils außerhalb der Nadeleinrichtung 105 angeordnet. In der mittleren Teildarstellung sind die Nadeln 700 innerhalb der Nadeleinrichtung 105 angeordnet.

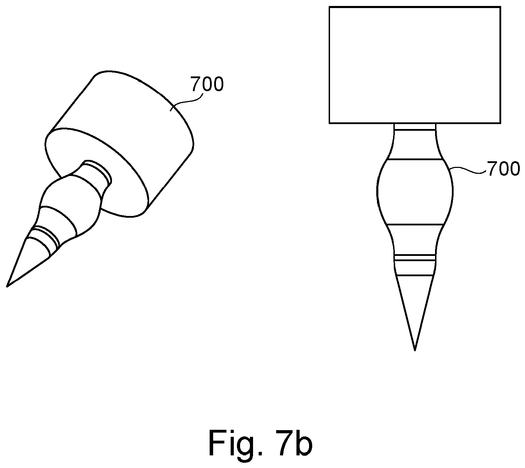

Figur 7b zeigt zwei Darstellungen von einem weiteren möglichen Ausführungsbeispiel für eine Geometrie der Nadeln 700. Während die Nadeln 700 in der Darstellung aus Figur 7a stabförmig oder gerade ausgeführt sind, ist aus der Figur 7b erkennbar, dass die Nadeln 700 auch bauchig oder mit einer Verdickung in einem Mittelteil der Nadeln 700 ausgeformt sein können. Hierdurch hat die Nadel 700 in der Mitte einen größeren Durchmesser als an einem Ende, sodass diese Verdickung dazu führt, dass eine derart ausgestaltete Nadel 700 nach einer Platzierung im oder am Ohr des Nutzers einen sehr guten Halt hat.

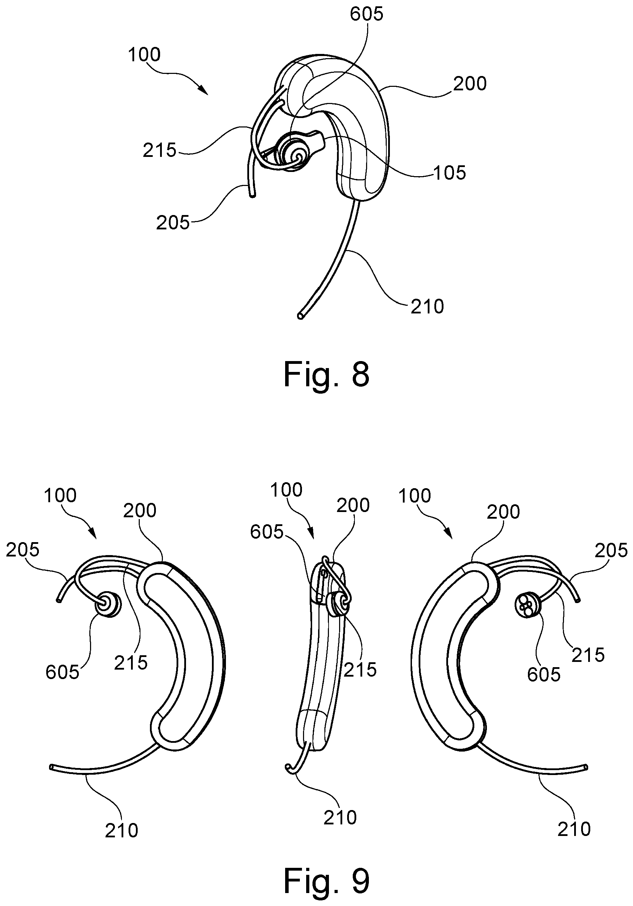

Figur 8 zeigt eine perspektivische Darstellung eines Ausführungsbeispiels einer Nervenstimulationsvorrichtung 100. Gezeigt sind die Nadeleinrichtung 105 und die Impulseinrichtung 200 als Merkmale der Nervenstimulationsvorrichtung 100. Die Nadeleinrichtung 105 und die Impulseinrichtung 200 sind über eine Verbindungsleitung 215 und über einen Magnetkontakt 605 miteinander verbunden.

Die Impulseinrichtung 200 umfasst ein Halteelement 205, unter dessen Verwendung sich die Impulseinrichtung 200 zum Beispiel an einem Ohr fixieren lässt. Darüber hinaus umfasst die Impulseinrichtung 200 eine Anschlussleitung 210 für einen Anschluss der Impulseinrichtung 200 beispielsweise an eine Recheneinheit.

Anders ausgedrückt zeigt Figur 8 eine Abbildung der hinter dem Ohr anzubringenden impulserzeugenden Vorrichtung mit den Magnetkontakten.

In anderen Worten zeigt Figur 8 eine magnetische Verbindung zwischen impulserzeugendem Gerät und festen Elektrodennadeln. Herkömmlicherweise kann eine Verbindung zwischen der Impulseinrichtung - auch impulsgebendes Gerät genannt - und der Nadeleinrichtung zum Beispiel durch kleine, miniaturisierte Steckverbindungen hergestellt werden. Dies kann jedoch für eine helfende Person schwierig sein und eine Selbstbedienung durch den Anwender verhindern. Eine technische Herausforderung bestand daher darin, eine einfache Verbindung zwischen der Nadeleinrichtung und der Impulseinrichtung zu ermöglichen. Gemäß einem Ausführungsbeispiel kann diese Herausforderung durch eine Verwendung insbesondere miniaturisierter Magnetkontakte gelöst werden. Das impulsgebende Gerät kann beispielsweise einen flexiblen Anschluss mit Magnetkontakten umfassen. Dieser flexible Anschluss kann dann mit Magnetkontakten der Nadeleinrichtung verbunden werden. Diese magnetische Verbindung zwischen der Impulseinrichtung und der Nadeleinrichtung kann dafür sorgen, dass diese beiden Einrichtungen in der richtigen Position und Ausrichtung verbunden werden, indem sie zum Beispiel einfach in die Nähe der jeweils anderen Einrichtung gebracht werden.

Figur 9 zeigt drei schematische Teildarstellungen jeweils eines Ausführungsbeispiels einer Nervenstimulationsvorrichtung 100 mit einer Impulseinrichtung 200. Die Impulseinrichtung 200 umfasst ein Halteelement 205, unter dessen Verwendung sich die Impulseinrichtung 200 zum Beispiel an einem Ohr fixieren lässt. Darüber hinaus umfasst die Impulseinrichtung 200 eine Anschlussleitung 210 für einen Anschluss der Impulseinrichtung 200 beispielsweise an eine Recheneinheit. Zudem umfasst die Impulseinrichtung 200 einen Magnetkontakt 605 zum Verbinden der Impulseinrichtung 200 mit einer nicht dargestellten Nadeleinrichtung. Die Impulseinrichtung 200 umfasst außerdem eine Verbindungsleitung 215 zum Verbinden der Impulseinrichtung 200 mit der in dieser Figur 9 nicht illustrierten Nadeleinrichtung.

Anders ausgedrückt zeigen die Teildarstellungen der Figur 9 Abbildungen der impulserzeugenden Vorrichtung, die hinter dem Ohr zu platzieren ist und die magnetischen Kontakte aufweist.

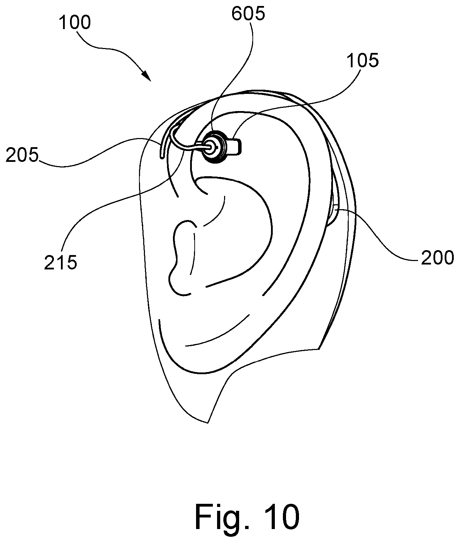

Figur 10 zeigt eine schematische Darstellung eines Ausführungsbeispiels einer Nervenstimulationsvorrichtung 100. Unter Verwendung der Nervenstimulationsvorrichtung 100 wird zumindest ein Nerv an einer Ohrmuschel elektrisch stimuliert, an welcher die Nadeleinrichtung 105 platziert ist. Eine Impulseinrichtung 200 ist unter Verwendung eines Halteelements 205 hinter der Ohrmuschel befestigt. Die Nadeleinrichtung 105 und die Impulseinrichtung 200 sind über eine Verbindungsleitung 215 und über einen Magnetkontakt 605 miteinander verbunden.

Anders ausgedrückt zeigt Figur 10 eine Visualisierung der magnetischen Verbindung zwischen den beiden Einheiten. Der Verbindungskopf der Baugruppe kann in die Nähe der vorpositionierten Nadeln gebracht werden. Die beiden eingebauten Magnete können helfen, die endgültige Position zu finden.

Gemäß einem Ausführungsbeispiel präsentiert der hier vorliegende Ansatz ein miniaturisiertes Gerät zur Ohmervenstimulation mit Manipulationsgriff zur Nadelfixierung und magnetischer Verbindung zwischen Nadeleinheit und impulserzeugender Vorrichtung.

Denkbar ist auch, dass die hier vorgestellte Nervenstimulationsvorrichtung in ein Hörgerät oder einen Kopfhörer integriert wird. Hierzu kann dann, was in den Figuren jedoch nicht explizit dargestellt ist, die Nervenstimulationsvorrichtung zumindest einen Lautsprecher aufweisen, über welchen Schall oder akustische Wellen an oder in das Ohr eines Nutzers dieser Nervenstimulationsvorrichtung ausgegeben werden. Ein solches Ausführungsbeispiel bietet den Vorteil, bekannte Gegenstände für einen Zusatznutzen weiterzuverwenden, sodass mit diesen Elementen ein Nutzerkomfort deutlich gesteigert werden kann. Dabei kann die Nervenstimulationsvorrichtung, die durch ein solches Hörgerät oder einen entsprechenden Kopfhörer gebildet wird, dann beispielsweise in 3 Modi betrieben werden, nämlich als reiner Lautsprecher (evtl. auch als Hörgerät), als reiner Stimulator (z. B. nachts wenn der Ton stört) oder im Kombibetrieb, wobei die Elektropulse einem bestimmten Takt folgen können, oder auch an eine Taktfrequenz einer Musik angepasst werden kann, also beispielsweise ein solcher Takt der elektrischen Impulse beschleunigt oder verlangsamt wird.

Gemäß dem vorliegenden Ansatz können obig genannte technische Herausforderungen, insbesondere die korrekte Positionierung der Nadeln im Bereich der Ohrmuschel bzw. des Ohrläppchens, durch eine geeignete Miniaturisierung einer Impulseinrichtung - alternativ als impulserzeugende Vorrichtung bezeichnet - im Besonderen in Kombination mit einer Nadelapplikationsvorrichtung, die es einem Anwender ermöglichen kann, die Nadeln an einer vorgesehenen Stelle des Ohrs zu applizieren, idealerweise überwunden oder verbessert werden. Darüber hinaus kann der vorliegende Ansatz eine einfache Verbindung der impulserzeugenden Vorrichtung mit den beispielsweise festen Nadeln über einen Magnetkontakt - auch magnetische Verbindung genannt - bieten, wodurch eine unter Umständen komplizierte Handhabung der Verbindung durch den Anwender oder eine helfende Person weitgehend vermieden werden kann. Somit können Ausführungsbeispiele gemäß dem vorliegenden Ansatz ausgeführter Geräte eine Selbstbedienung durch den Anwender ermöglichen, was einen Einsatz der Geräte in der häuslichen Pflege ermöglichen kann. Gleichzeitig können die Miniaturisierung und eine einfache Handhabung - speziell die Positionierung, eine Fixierung und die Verbindung der Nadeln mit der impulsgebenden Vorrichtung - auch für eine Verwendung der Vorrichtung in einer klinischen Umgebung von Vorteil sein.

Herkömmliche Nervenstimulationsvorrichtungen können in der Lage sein, elektrische Energie an die Ohrmuschelregion abzugeben. Der hier dargelegte Ansatz betrifft eine Nervenstimulationsvorrichtung zur elektrischen Stimulation der Ohrmuschel. Eine solche Nervenstimulationsvorrichtung kann beispielsweise zur Verbesserung oder Behandlung einer Reihe von medizinischen und nicht-medizinischen Zuständen verwendet werden. Insbesondere betrifft der hier dargelegte Ansatz eine Kombination aus einem miniaturisierten impulserzeugenden Gerät mit einer Nadeleinheit, die lediglich beispielhaft über Magnetkontakte verbunden werden kann. Gemäß einem Ausführungsbeispiel kann die Nadeleinheit einen abnehmbaren Griff zur Fixierung im Ohr enthalten. Ausführungsbeispiele der Nervenstimulationsvorrichtung können zum Beispiel zur Linderung von Muskelverspannungen, zur Schmerzlinderung, bei peripheren arteriellen Gefäßerkrankungen, bei entzündlichen Zuständen oder bei Vorhofflimmern eingesetzt werden.

Der offenbarte Ansatz bezieht sich mit anderen Worten auf ein elektrisches und/oder neurales Stimulationsgerät. Insbesondere bezieht sich der offenbarte Ansatz auf ein Gerät zur elektrischen Stimulation der Ohrmuschel und/oder des Ohrläppchens. Ein derartiges Gerät kann zur Verbesserung oder Behandlung einer Reihe medizinischer und nicht-medizinischer Zustände verwendet werden.

Abgesehen von der spezifischen Stimulation des Vagusnervs oder anderer Nerven kann die aurikuläre Elektrostimulation beispielsweise bei medizinischen und nicht-medizinischen Erkrankungen eingesetzt werden. Die Nerven im Außenohr sind üblicherweise rein afferenten (sensorischen) Ursprungs. Das würde bedeuten, dass die Impulse ausschließlich oder fast ausschließlich an den Hirnstamm gerichtet sind. Herkömmlicherweise sollte es keine Rolle spielen, welche Nerven am Außenohr stimuliert werden, da sie im Normalfall alle an den *Nucleus tractus solitarii,* also an den neuronalen Geschmackskern, andocken. Es kann jedoch wichtig sein, nahe am Nerv zu stimulieren, da typischerweise nur eine sanfte Stimulation Axone zur Weiterleitung von Impulsen anregt. Es sei an dieser Stelle darauf hingewiesen, dass eine Oberflächenstimulation oftmals nicht für eine Langzeitstimulation geeignet ist, da die Nervenenden sehr schnell refraktär werden können. Die Fossa triangularis, also das Außenohr, kann ein idealer Ort für eine solche Stimulation sein, da sich in einem Bereich der Fossa triangularis üblicherweise ein dichtes Nervengeflecht des großen Ohrastes befindet. Darüber hinaus kann eine Vertiefung der Fossa triangularis ein Aufliegen eines Objekts erleichtern. Die Fossa triangularis weist typischerweise ein dichtes Nervengeflecht auf.

Ein weiterer Aspekt eines Wirkungsmechanismus kann mit einer Art der Stimulation zusammenhängen. Es sei daran erinnert, dass es bei einer Überstimulation der Nerven, wie sie bei einer Rückenmarkstimulation auftritt, zu einer Blockade der Reizleitung kommen kann, und dass die Nervenleitung refraktär werden kann. In der Schmerztherapie kann dies erfolgreich zu einer Schmerzblockade eingesetzt werden. Im Gegensatz dazu kann bei einer aurikulären "Low-Level-Neurostimulation" der afferente Nerv als Transporteur von Erregungspotentialen zum Hirnstamm fungieren. Dadurch können beispielhaft Regulationsprozesse im Hirnstamm stimuliert werden. Bei fortgesetzter intermittierender Stimulation kann dies zu einer Modulation von Neuronen und/oder Synapsen und damit zu einer Verbesserung wichtiger Regulationsmechanismen führen.

Während eine Anwendung von TENS, zum Beispiel bei einer Schmerzbehandlung, häufig von Patienten oder Anwendern selbst vorgenommen wird, wird die Anwendung von PENS üblicherweise häufiger von medizinischem Personal und/oder Praktikern durchgeführt.

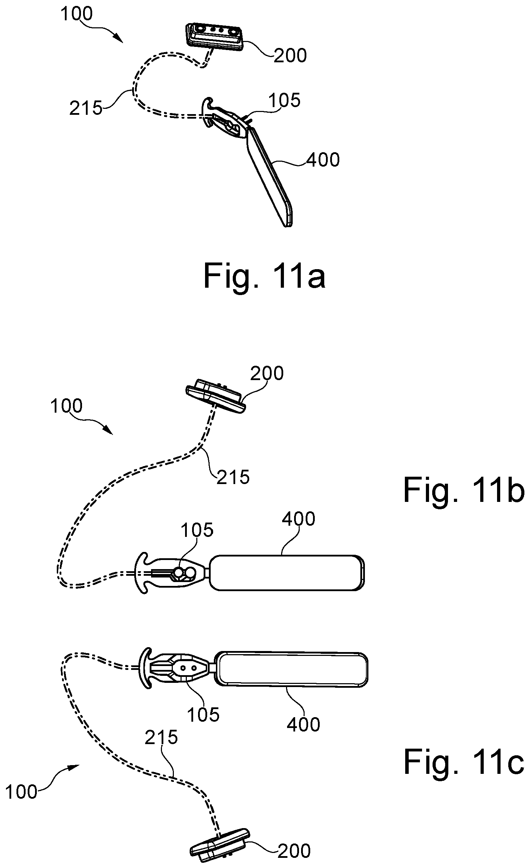

Figur 11a zeigt eine perspektivische Ansicht einer Nervenstimulationsvorrichtung 100 gemäß einem Ausführungsbeispiel des hier vorgestellten Ansatzes. Hierbei ist erkennbar, dass die Impulseinrichtung 200 mittels der Verbindungsleitung 215 mit der Nadeleinrichtung 105 gekoppelt ist. Anzumerken ist hierbei, dass die durchgezogenen Linien Teile der Nervenstimulationsvorrichtung 100 abbilden, die beispielsweise für die Funktionalität oder den Einsatz der hier vorgestellte Nervenstimulationsvorrichtung 100 wichtig sind, wogegen Teile, welche durch gestrichelte Linien dargestellt sind, für die Funktionalität des hier vorgestellten Ansatzes weniger relevant sind. Dennoch ist anzumerken, dass nicht zwingend alle mit durchgezogenen Linien dargestellten Elemente auch für die Funktion des hier vorgestellten Ansatzes notwendig sind.

Figur 11b zeigt eine frontale Ansicht der Nervenstimulationsvorrichtung 100 gemäß einem hier vorgestellten Ausführungsbeispiel. Erkennbar ist wiederum die Impulseinrichtung 200, welche über die Verbindungsleitung 215 mit der Nadeleinrichtung 105 verbunden ist, welche selbst wieder durch einen Griff bzw. eine Griffeinrichtung 400 zur besseren Platzierung am Ohr eines Nutzers aufgenommen ist.

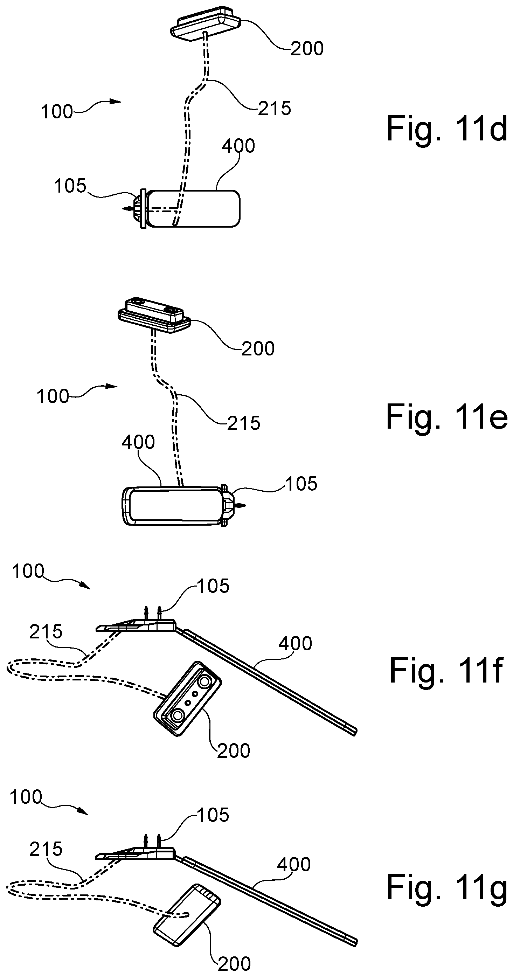

Figur 11c zeigt eine rückseitige Ansicht eines Ausführungsbeispiels der Nervenstimulationsvorrichtung 100, wobei ebenfalls wiederum die in den vorstehend genannten Passagen zu Teilfigur und Figur 11 genannten Elemente abgebildet sind.

Figur 11d zeigt eine Seitenansicht eines Ausführungsbeispiels der Nervenstimulationsvorrichtung 100 von einer linken Seite.

Figur 11e zeigt eine Seitenansicht eines Ausführungsbeispiels der Nervenstimulationsvorrichtung 100 von einer rechten Seite.

Figur 11f zeigt eine Draufsicht auf ein Ausführungsbeispiel der Nervenstimulationsvorrichtung 100.

Figur 11g zeigt eine Ansicht auf ein Ausführungsbeispiel der Nervenstimulationsvorrichtung 100 von unten.

In den Teilfiguren der Figur 11 ist somit der erkennbar, dass die Impulseinrichtung 200 flexibel durch die Verbindungsleitung 215 mit der Nadeleinrichtung 105 verbunden ist, wobei zur besseren Positionierung die Nadeleinrichtung 105 durch die Griffeinrichtung 400 gehalten wird. Denkbar ist auch, dass die Nadeleinrichtung 105 von der Verbindungsleitung 215 und/oder der Impulseinheit 200 getrennt werden kann, beispielsweise um eine bessere Positionierung jedes der einzelnen Komponenten der Nervenstimulationsvorrichtung 100 vornehmen zu können und nachfolgend beispielsweise die beiden Komponenten wieder miteinander zu verbinden. Auf diese Weise lässt sich eine komfortable Anbringung der eitlen Komponenten der Nervenstimulationsvorrichtung 100 realisieren. Denkbar ist auch, dass einzelne Komponenten beispielsweise mittels Magneten miteinander gekoppelt werden, sodass ein sicherer Halt am Nutzer der Nervenstimulationsvorrichtung 100 und eine möglichst geringe Verletzung von Körperteilen des Nutzers durch die Anbringung der Nervenstimulus uns Vorrichtung 100 erreicht werden kann.

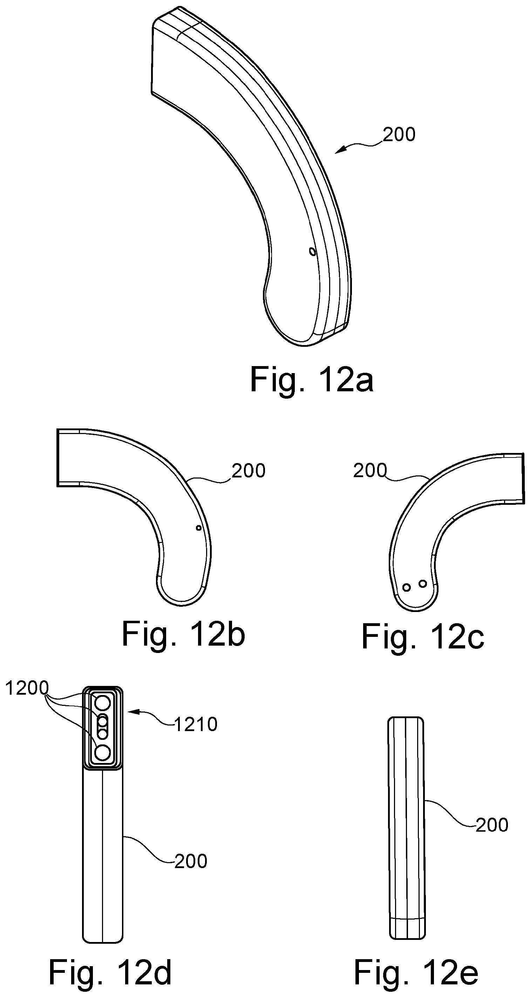

Figur 12a zeigt eine perspektivische Ansicht eines Ausführungsbeispiels der Impulseinrichtung 200. Hierbei ist erkennbar, dass die Impulseinrichtung 200 gebogen oder C-förmig ausgestaltet werden kann, um besonders günstig beispielsweise hinter einem Ohr eines Nutzers der Nervenstimulationsvorrichtung platziert zu werden. Auf diese Weise ist auch die Benutzung der Nervenstimulationsvorrichtung durch den Nutzer weniger offensichtlich, da die Impulseinrichtung 200 in diesem Fall beispielsweise durch einen Teil des Ohrs des Nutzers der Impulseinrichtung 200 bzw. der Nervenstimulationsvorrichtung, dessen Teil diese Impulseinrichtung 200 ist.

Figur 12b zeigt eine Frontansicht eines Ausführungsbeispiels der Impulseinrichtung 200.

Figur 12c zeigt eine rückseitige Ansicht eines Ausführungsbeispiels der Impulseinrichtung 200.

Figur 12d zeigt eine Seitenansicht eines Ausführungsbeispiels der Impulseinrichtung 200 von einer linken Seite. Hierbei sind verschiedene Anschlüsse 1200 oder Schalter erkennbar, die in einem Anschlussbereich 1210 der Impulseinrichtung 200 angeordnet sind und mittels welchen beispielsweise die Funktion der Impulseinrichtung 200 gesteuert werden kann.

Figur 12e zeigt eine Seitenansicht eines Ausführungsbeispiels der Impulseinrichtung 200 von einer rechten Seite.

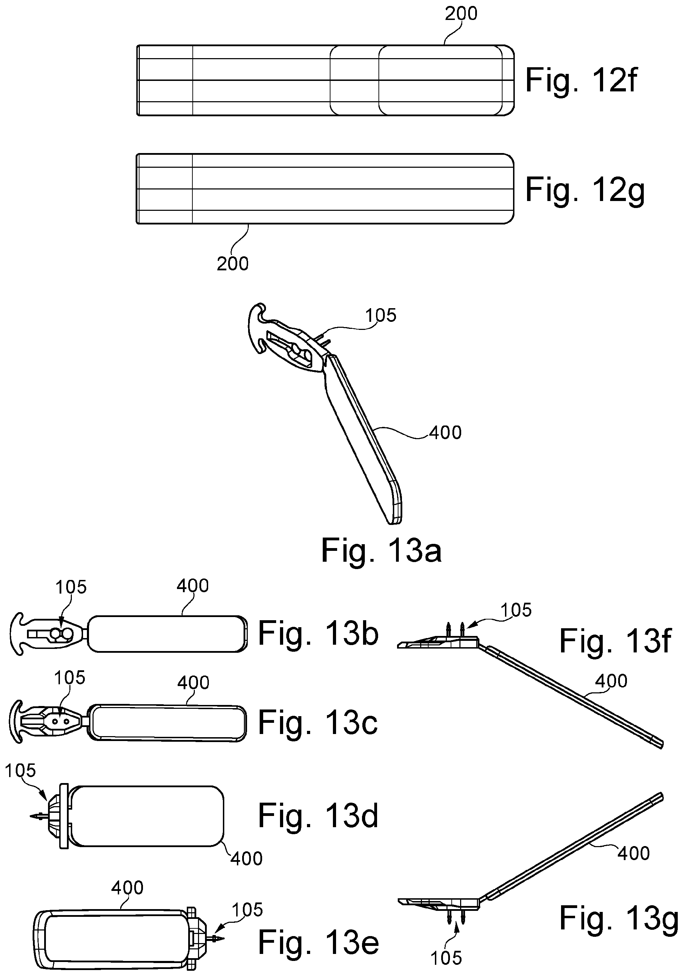

Figur 12f zeigt eine Draufsicht auf ein Ausführungsbeispiel der Impulseinrichtung 200.

Figur 12g zeigt eine Ansicht eines Ausführungsbeispiels der Impulseinrichtung 200 von unten.

Figur 13a zeigt ein Ausführungsbeispiel der Griffeinrichtung 400 mit einer daran befestigten Nadeleinrichtung 105. Erkennbar ist hierbei, dass die Griffeinrichtung 400 im Bereich der Nadeleinrichtung 105 abgedeckt ist, um eine genauere bzw. einfachere Positionierung der Nadeleinrichtung 105 an einem Ohr eines Nutzers der hier vorgestellten Nervenstimulationsvorrichtung zu ermöglichen.

Figur 13 b zeigt eine Frontansicht eines Ausführungsbeispiels der Griffeinrichtung 400 mit einer daran befestigten Nadeleinrichtung 105.

Figur 13c zeigt eine Ansicht eines Ausführungsbeispiels der Griffeinrichtung 400 mit einer daran befestigten Nadeleinrichtung 105 von einer Rückseite.

Figur 13d zeigt eine Ansicht eines Ausführungsbeispiels der Griffeinrichtung 400 mit einer daran angebrachten Nadeleinrichtung 105 von einer linken Seite.

Figur 13e zeigt eine Ansicht eines Ausführungsbeispiels der Griffeinrichtung 400 mit einer daran angebrachten Nadeleinrichtung 105 von einer rechten Seite.

Figur 13f zeigt eine Ansicht eines Ausführungsbeispiels der Griffeinrichtung 400 mit einer daran angebrachten Nadeleinrichtung 105 von oben.

Figur 13g zeigt eine Ansicht des Ausführungsbeispiels der Griffeinrichtung 400 mit einer daran befestigten Nadeleinrichtung 105 von unten.

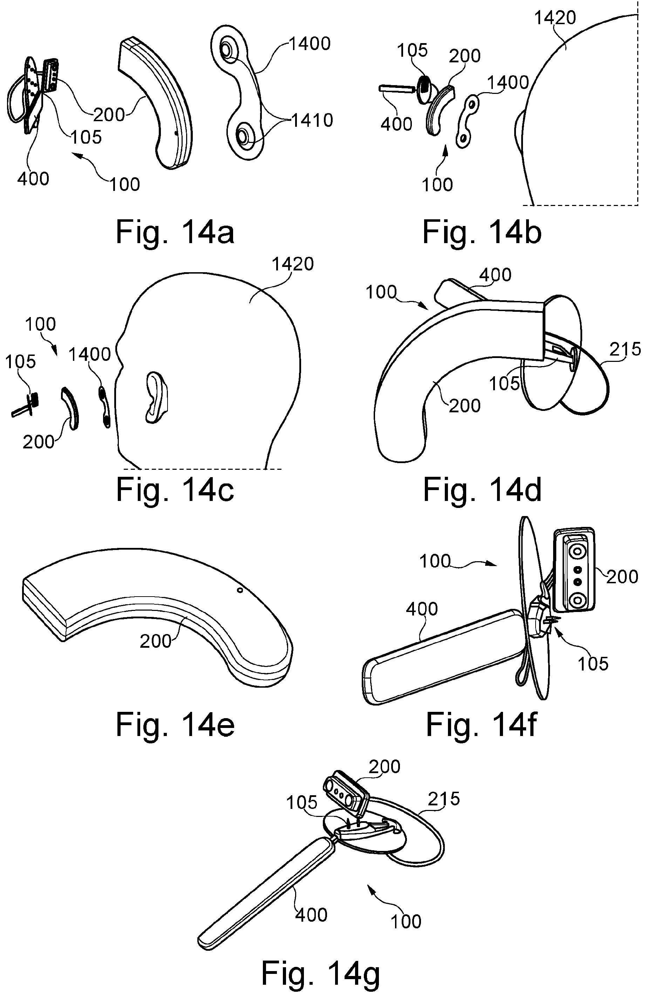

Figur 14a zeigt eine perspektivische Darstellung einer Nervenstimulationsvorrichtung 100 gemäß einem Ausführungsbeispiel. bei dieser Nerven stimuliert uns Vorrichtung 100 ist weiterhin beispielsweise ein Kopfpflaster 1400 vorgesehen, welches beispielsweise Magnete 1410 umfasst, welche mit entsprechenden, in der Figur 14a nicht dargestellten Magneten der Impulseinrichtung 200 zusammenwirken können, sodass die Impulseinrichtung 200 beispielsweise an einer gewünschten Position an einem Kopf eines Nutzers der Nervenstimulationsvorrichtung 100 sicher fixiert werden kann.

Figur 14b zeigt eine Explosionsdarstellung eines Ausführungsbeispiels der Nervenstimulationsvorrichtung 100, wobei nun erkennbar ist, wie das Kopfpflaster 1400 an oder hinter einem Ohr am Kopf 1420 des Nutzers der Nervenstimulationsvorrichtung 100 befestigt werden kann.

Figur 14c zeigt eine weitere Möglichkeit zur Anordnung oder Befestigung des Kopfpflasters 1400 am Kopf 1420 des Nutzers, nun aus einer anderen Perspektive.

Figur 14d zeigt eine perspektivische Darstellung eines Ausführungsbeispiels der Nervenstimulationsvorrichtung 100.

Figur 14e zeigt eine weitere perspektivische Darstellung einer Impulseinrichtung 200, die als Teil der hier vorgestellten Nervenstimulationsvorrichtung 100 gemäß einem Ausführungsbeispiel verwendet werden kann.

Figur 14f zeigt eine perspektivische Darstellung eines weiteren Ausführungsbeispiels der Nervenstimulationsvorrichtung 100.

Figur 14g zeigt eine perspektivische Darstellung eines weiteren Ausführungsbeispiels der Nervenstimulationsvorrichtung 100.

Weiterhin können bereits auch Einsatz-Erfahrungen mit der hier vorgestellten Ansatz näher angeführt werden. wie bekannt ist, ist unser menschlicher Hirnstamm der Regulator, der für alle Veränderungen Informationen aus dem gesamten Körper benötigt wird, auch aus dem Bereich des Denkens, Sehens, Hörens, Fühlens usw. Neu in der Hypothese ist dabei, dass beispielsweise ein Älterwerden oder eine Art chronischer Krankheit und eine Art von Stress, die Anforderungen von der Peripherie zu niedrig werden und nach einer Weile ist dies für den Hirnstamm normal und kann nicht mehr reguliert werden. Dabei ist wichtig, dass wenn die Stimulation von irgendwoher ausgeht, beim Gehen, bei jeder Sportart, beim Sprechen usw., sich die Sensorik des Empfängers im Hirnstamm verbessert.

Die Stimulation in Form von Bewegung, Lauftraining, Muskeltraining hat beide Effekte. Der Körper wird trainiert und auch der Empfänger im Hirnstamm verbessert sich.

Zu viele und zu starke sensorische Impulse machen die Sensorik refraktär und die Impulse an den Empfänger im Hirnstamm werden blockiert. Es ist wie ein Schutz für den Hirnstamm. Zum Beispiel lassen wir zu viel Werbung durch.

Der Vagusnerv ist zu 80 % afferent und leitet Informationen vom gesamten Rumpf zum Hirnstamm.

Die efferenten Teile des Vagusnervs geben Befehle entweder an den Sympathikus oder an den Parasympathikus weiter und er reguliert den Blutfluss überall und alle Entzündungen (Zytokin-Regulierung).

Das gesamte Außenohr hat nur sensorische Nerven und im Bereich der Fossa triangularis ist die Nervenstruktur so eng, dass man mit der hier vorgestellten Doppelnadel bzw. der Nadeleinrichtung 105 den Nerv nicht verfehlen kann. Der Akupunkturarzt kann sie beispielsweise auch verwenden, weil er den Nerv immer trifft. Aber die Nervenenden sind sehr empfindlich und nach kurzer Zeit wird die Auslösung blockiert.

Mit der hier vorgestellten Doppelnadel bzw. der Nadeleinrichtung 105 kann die Nervenstruktur tiefer und mit sehr geringer Intensität und niedriger Frequenz und mit Pausen stimuliert werden. Auf diese Weise kann ein Trainingseffekt für den Empfänger im Hirnstamm bei Tag und bei Nacht erreicht werden. Hierbei ist zu bedenken, dass ein gut funktionierender Hirnstamm die Situation nicht reparieren kann. Ein trainierter Hirnstamm kann auch nur das Maximum erreichen. Der Körper braucht Hilfe von außen.

Was hierbei angeführt werden kann, ist die Tatsache, dass die Hirnstammstimulation oder die sensorische Vagusstimulation, das ist eine Frage der Nomenklatur, immer dazu beiträgt, den Empfänger zu trainieren. Jede Entzündung kann Schmerzen verursachen. Normalerweise wird der Hirnstamm darüber informiert und repariert diese Entzündungen. Bei chronischen Schmerzen erkennt er sie als normal und repariert sie nicht. Mit dem hier vorgestellten Neurostimulator können zum Beispiel bei einem Rückenmarkstumor überdurchschnittliche Erfolgsquote erreicht werden.

Durch intelligente Stromstimulation des Gehirns können Neuronen und Synapsen vermehrt werden. Er konnte die Plastizität des Gehirns zeigen. Das Gehirn braucht hierbei oftmals Informationen, sowohl vom Körper als auch als Gedächtnis für die Regulierung. Es braucht die sensorischen Bahnen.

Wenn es weniger Informationen erhält, wird es die Regulierung einstellen. Dies kann beispielsweise in folgenden Fällen auftreten.
- PAD - Periphere arterielle Verschlusskrankheit
- Die Arteriolen haben die Aufgabe, das Blut überall hin im Körper zu bringen
- Flüssigkeiten wie Blut würden den kürzesten Weg nehmen und sich nicht ausbreiten. Die Arteriolen und Venolen können das Blut überall hin pumpen. Dazu ist es notwendig, dass der Hirnstamm, unser Regulator, Informationen erhält.

Wenn die Arterien stärker verkalken, gelangt weniger Blut in die Peripherie. Die Nerven werden schwächer. Mit der Zeit sieht der Hirnstamm dies als normal an und reduziert seine Aktivität.

Wenn die großen Gefäße nicht behandelt werden können, ist das Gehen die einzige Möglichkeit, Signale an den Hirnstamm zu senden. Viele Menschen haben das Problem, dass das Gehen schmerzhaft ist.

Der hier vorgestellte Stimulator kann mit niedriger Stimulationsstufe die Sensibilität im menschlichen Hirnstamm erhöhen und der Hirnstamm erkennt die geringeren Anfragen aus der Peripherie. Danach wird er die Vasomotion wieder starten.

Der erhöhte Blutfluss kann manchmal nach einer Stunde gemessen werden.

Hieraus kann als Fazit gezogen werden: Es ist möglich, den sensorischen Pfad durch intelligente Stimulation zu trainieren. Weniger Leistung und Pausen sind wichtig, sonst wird das System refraktär. Mit zunehmendem Alter ist die Stimulation wie ein Jungbrunnen.

Grundsätzlich wird die Sensorik des menschlichen Hirnstamms immer einen Nutzen haben.

Wenn der Hirnstamm sie nicht reparieren kann wird die Funktion des hier vorgestellten Ansatzes jedoch schwierig. Wenn die Arteriolen und Venolen zu stark verkalkt sind und ihre Flexibilität verloren haben, kann der Hirnstamm die Verkalkung nicht oder nur ungenügend beseitigen.

Wenn die Nerven in der Peripherie fast abgestorben sind, kann der Hirnstamm die Nerven nicht wiederbeleben. Es kann in diesem Fall nur ein Versuch unternommen werden. Beispielsweise kann die Amputation eines Drittels der kritischen Ischämie verhindern.

Ein weiterer Punkt des Wirkmechanismus des hier vorgestellten Ansatzes ist die Regulierung von Zytokinen. Die Stimulation wird die Sensorik im Hirnstamm wieder erhöhen, die bessere Regulierung von Zytokinen reduziert Entzündungen und Schmerzen werden reduziert.

Kopfschmerzen sind, wie bekannt ist, ein weit verbreitetes Leiden. Doch oft ist die Ursache unbekannt. Wenn die Ursache nicht bekannt ist, kann man nur die Stimulationsbehandlung versuchen. Hierzu sollte jedoch die Sensorik trainiert werden. Das bedeutet oftmals bis zu 3-wöchige Behandlungen. Eine Behandlung über 3 Wochen sollte erfolgen, weil eine Entzündung etwa 3 Wochen braucht, um zu heilen. Wenn die Ursache eine Verspannung ist, sollte man mit Dehnübungen den Behandlungserfolg unterstützen. Die Kommunikation zwischen Hirnstamm und peripheren Informationen sollte immer möglich sein. Die Bedeutung der Sinneswahrnehmung wird beispielsweise im Vagusnerv erkannt. Der Vagusnerv hat 80 % afferente und nur 20 % efferente Bahnen. Der Regulator ist jedoch immer der Hirnstamm und nicht der Vagus.

Insgesamt kann angemerkt werden, dass seit 2004 Erfahrung mit verschiedenen Arten von Stimulationsbehandlungen vorhanden ist. Manchmal konnten große Erfolge und manchmal weniger oder keinen Erfolg bei einer entsprechenden Behandlung erkannt werden.

Nach diesen Erfahrungen wurde als Hypothese aufgestellt, dass der menschliche Hirnstamm der Regulator für alle Veränderungen ist und er Informationen aus dem ganzen Körper benötigt, auch aus dem Denken, Sehen, Hören, Fühlen und so weiter. Neu an der Hypothese ist, dass mit zunehmendem Alter oder bei chronischen Krankheiten und bei Stress die Anforderungen aus der Peripherie zu gering werden und nach einer Weile das für den Hirnstamm so normal ist, dass er es nicht mehr regulieren kann. Wichtig ist dabei, dass die Stimulation von irgendwoher kommt, beim Gehen, beim Sport, beim Sprechen und so weiter, sodass sich sich die Sensorik des Empfängers im Hirnstamm verbessert. Die Stimulation in Form von Bewegung, Laufen, Muskeltraining hat beide Effekte. Der Körper wird trainiert und auch der Empfänger im Hirnstamm verbessert sich. Zu viele und zu starke sensorische Impulse machen die Sensorik refraktär und die Impulse zum Empfänger im Hirnstamm werden blockiert. Es ist wie ein Schutz für den Hirnstamm. Wenn zum Beispiel zu viel Werbung gehört wird, lassen wir sie passieren. Der Vagusnerv ist zu 80% afferent und bringt Informationen aus dem gesamten Rumpf zum Hirnstamm. Die efferenten Anteile des Vagusnervs geben entweder dem Sympartikus oder dem Parasympathikus Befehle.

Das ganze Außenohr hat nur sensorische Nerven und im Bereich der Fossa triangularis ist die Nervenstruktur so dicht, dass man mit der hier vorgestellten Doppelnadel bzw. der Nadeleinrichtung 105 den Nerv nicht verfehlen kann. Der Ohrakupunkturarzt benutzt sie auch mit, weil er den Nerv immer trifft. Aber die Enden der Nerven sind sehr empfindlich und nach kurzer Zeit wird die Auslösung blockiert. Mit der hier vorgestellten Doppelnadel bzw. Nadeleinrichtung 105 kann die Nervenstruktur tiefer und mit sehr geringer Intensität und niedriger Frequenz und mit Pausen stimuliert werden. So wird Tag und Nacht ein Trainingseffekt für den Empfänger im Hirnstamm erreicht. Zu Bedenken ist, dass wenn ein gut funktionierender Hirnstamm die Situation nicht reparieren kann, kann ein zurückgebliebener Hirnstamm auch nur das Maximum erreichen. Der Körper braucht Hilfe von außen. Was gesagt werden kann, ist, dass Hirnstammstimulation oder sensorische Vagusstimulation, es ist eine Frage der Nomenklatur, immer hilft, den Empfänger zu trainieren. Jede Entzündung kann Schmerzen verursachen. Normalerweise wird der Hirnstamm darüber informiert und er repariert ihn. Bei chronischen Schmerzen erkennt er sie als normal an und repariert sie nicht.

Ferner kann der hier vorgestellte Ansatz auch im Bereichg des Cervical and Low back pain (Schmerzen in der Halswirbelsäule und im unteren Rücken) eingesetzt werden. Eine Studie hatte hierzu ein großartiges Ergebnis, aber sie hatte das Wort Akupunktur im Titel, und das Wort Akupunktur ist in der wissenschaftlichen Medizin nicht akzeptiert, speziell wurden keine Doppelblindstudien gemacht.

Als Schlussfolgerung kann nun gezogen werden, dass durch intelligente Stromstimulation des Gehirns Neuronen und Synapsen vermehrt werden konnten. Der hier vorgestellte Ansatz konnte somit die Plastizität des Gehirns zeigen. Hieraus lässt sich als wichtigste Erkenntnis ziehen, dass das Gehirn bzw. der Hirnstamm immer wieder Informationen aus unserem Körper als Gedächtnis zur Regulation benötigt. Es/Er braucht die sensorischen Bahnen. Wenn es/er weniger Informationen bekommt, wird er die Regulation herunterfahren.

Anzumerken ist, dass die Flüssigkeit wie Blut den kürzesten Weg nehmen würde, um sich im Körper auszubreiten. Die Arteriolen und Venolen können das Blut überall hinpumpen. Dazu ist es notwendig, dass der Hirnstamm, unser Regulator, Informationen erhält. Wenn die Arterien immer mehr verkalken, gelangt weniger Blut in die Peripherie. Die Nerven werden schwächer. Mit der Zeit sieht der Hirnstamm das als normal an und er reduziert seine Aktivität. Der Teufelskreis beginnt.

Wenn die großen Gefäße nicht behandelt werden können, ist Gehen die einzige Möglichkeit, Signale an den Hirnstamm zu senden. Viele Menschen haben das Problem, dass das Gehen schmerzhaft ist. Der hier vorgestellte Stimulator kann mit schwacher Stimulation die Sensibilität in unserem Hirnstamm erhöhen und der Hirnstamm wird die geringeren Anfragen aus der Peripherie erkennen. Danach wird er die Vasomotion wieder in Gang setzen. Der erhöhte Blutfluss kann manchmal nach einer Stunde gemessen werden.

Als weitere Schlussfolgerung kann gezogen werden, dass es möglich ist, die sensorischen Bahnen über intelligente Stimulation zu trainieren. Weniger Strom und Pausen sind wichtig, sonst wird das System refraktär. Wenn man älter wird, ist die Stimulation wie ein Jungbrunnen. Im Grunde genommen wird die Sensorik unseres Hirnstamms immer einen Nutzen haben.

## Patentansprüche

1. Nadeleinrichtung (105), die ausgebildet ist, um an der Ohrmuschel und/oder am Ohrläppchen platziert oder platzierbar zu sein, wobei die Nadeleinrichtung (105) eine Griffeinrichtung (400) und vorzugsweise eine Platzierungseinheit (400) zum Platzieren der Nadeleinrichtung (105) an der Ohrmuschel und/oder am Ohrläppchen aufweist. wobei die Nadeleinrichtung (105) eine Sollbruchstelle (600) zum Trennen der Griffeinrichtung (400) von der Nadeleinrichtung (105) aufweist und vorzugsweise wobei die Platzierungseinrichtung (400) der Nadeneinrichtung (105) als Pinzette oder als Klammer ausgebildet ist und vorzugsweise wobei die Platzierungseinrichtung (400) der Nadeneinrichtung (105) ein Schnappverschlusselement und/oder ein Klickverschlusselement und/oder ein Magnetverschlusselement zum zerstörungsfreien Trennen von Nadeln (700) der Nadeleinrichtung (105) von der Platzierungseinrichtung (400) aufweist.

2. Nadeleinrichtung (105) gemäß Anspruch 1, wobei die Nadeleinrichtung (105) mittels zumindest eines Magnetkontakts (605) mit einer Impulseinrichtung (200) miteinander trennbar verbunden oder verbindbar ist, wobei die Impulseinrichtung (200) mit der Nadeleinrichtung (105) zur Abgabe zumindest eines elektrischen Impulses an die Ohrmuschel und/oder an das Ohrläppchen koppelbar oder gekoppelt ist und/oder wobei die Nadeleinrichtung (105) mittels zumindest einer Klammer oder Pinzette mit der Impulseinrichtung (200) trennbar verbunden oder verbindbar ist.

3. Nadeleinrichtung (105) gemäß einem der vorangehenden Ansprüche, wobei die Nadeleinrichtung (105) als zumindest eine Elektrodennadel und/oder als zumindest eine Mikronadel und/oder als Nadelelement mit zumindest zwei Nadeln ausgeformt ist, insbesondere wobei die zumindest eine Elektrodennadel und/oder die zumindest eine Mikronadel aus einem biokompatiblen Material, insbesondere aus einem biokompatiblen Metall, ausgeformt ist oder sind.

4. Nadeleinrichtung (105) gemäß einem der vorangehenden Ansprüche, wobei die Nadeleinrichtung (105) zumindest ein Klebeelement (405) zum Befestigen der Nadeleinrichtung (105) an der Ohrmuschel und/oder am Ohrläppchen aufweist.

5. Nervenstimulationsvorrichtung (100) zum elektrischen Stimulieren zumindest eines Nervs zumindest einer Ohrmuschel und/oder zumindest eines Ohrläppchens, wobei die Nervenstimulationsvorrichtung (100) die folgenden Merkmale aufweist:
- eine Nadeleinrichtung (105) gemäß einem der Ansprüche 1 bis 4; und
- eine von der Nadeleinrichtung (105) trennbare Impulseinrichtung (200) zur Erzeugung von elektrischen Impulsen, wobei die Impulseinrichtung (200) mit der Nadeleinrichtung (105) zur Abgabe zumindest eines elektrischen Impulses an die Ohrmuschel und/oder an das Ohrläppchen koppelbar oder gekoppelt ist.

6. Nervenstimulationsvorrichtung (100) gemäß Anspruch 5, wobei die Impulseinrichtung (200) ausgebildet ist, um elektrische Impulse in einem Stromstärkenbereich zwischen 0,05 Milliampere und 1,2 Milliampere abzugeben.

7. Nervenstimulationsvorrichtung (100) gemäß einem der vorangehenden Ansprüche 5 oder 6, wobei die Impulseinrichtung (200) ausgebildet ist, um elektrische Impulse in einem Toleranzbereich um einen Frequenzwert von 1 Hertz abzugeben.

8. Nervenstimulationsvorrichtung (100) gemäß einem der vorangehenden Ansprüche 5 bis 7, wobei die Impulseinrichtung (200) zumindest ein Halteelement (205, 210) zum Fixieren der Impulseinrichtung (200) am Ohr aufweist.

9. Nervenstimulationsvorrichtung (100) gemäß einem der vorangehenden Ansprüche 5 bis 8, wobei die Impulseinrichtung (200) mit elektrischer Energie aufladbar ist.

10. Nervenstimulationsvorrichtung (100) gemäß einem der vorangehenden Ansprüche 5 bis 9, wobei die Impulseinrichtung (200) ausgebildet ist, um unter Verwendung einer Recheneinheit gesteuert oder steuerbar zu sein und/oder wobei ein Steuerprogramm der Impulseinrichtung (200) veränderbar ist.

11. Nervenstimulationsvorrichtung (100) gemäß einem der vorangehenden Ansprüche 5 bis 10, wobei die Impulseinrichtung (200) ausgebildet ist, um eine Stimulation zumindest eines Ohrnervs am Außenohr und/oder eine Stimulation des Vagusnervs auszuführen.

12. Nervenstimulationsvorrichtung (100) gemäß einem der vorangehenden Ansprüche 5 bis 1, wobei die Impulseinrichtung (200) ausgebildet ist, um hinter der Ohrmuschel befestigt zu werden und/oder wobei die Nervenstimulationsvorrichtung (100) zusätzlich mit einem Lautsprecher ausgestattet ist, insbesondere wobei dieser Lautsprecher an oder in der Nadelvorrichtung (105) und/oder der Impulsvorrichtung (200) angebracht wird und/oder wobei die Nervenstimulationsvorrichtung (100) zusätzlich mit einem Lautsprecher und/oder ergänzt durch einen in einem Ohr angeordneten Kopfhörer ausgestattet ist.

## Claims

1. A needle device (105) configured to be placed or placeable on the auricle and/or the earlobe, wherein the needle device (105) comprises a gripping element (400) and/or a placement element (400) for placing the needle device (105) on the auricle and/or the earlobe, wherein the needle device (105) comprises a predetermined breaking point (600) for separating the gripping element (400) from the needle device (105), and/or wherein the placement element (400) of the needle device (105) is configured as tweezers and/or as a clip, and/or wherein the placement element (400) of the needle device (105) comprises a snap-fit fastening element and/or a click-lock fastening element and/or a magnetic fastening element for non-destructively separating needles (700) of the needle device (105) from the placement element (400).

2. The needle device (105) according to claim 1, wherein the needle device (105) is detachably connected or connectable to a pulse generator (200) by means of at least one magnetic contact (605), wherein the pulse generator (200) is or can be coupled to the needle device (105) for delivering at least one electrical pulse to the auricle and/or the earlobe, and/or wherein the needle device (105) is detachably connected or connectable to the pulse generator (200) by means of at least one clip or tweezers.

3. The needle device (105) according to any one of the preceding claims, wherein the needle device (105) is configured as at least one electrode needle and/or at least one microneedle and/or as a needle element comprising at least two needles, wherein, in particular, the at least one electrode needle and/or the at least one microneedle is made of a biocompatible material, in particular a biocompatible metal.

4. The needle device (105) according to any one of the preceding claims, wherein the needle device (105) comprises at least one adhesive element (405) for attaching the needle device (105) to the auricle and/or the earlobe.

5. A nerve stimulation device (100) for electrically stimulating at least one nerve of at least one auricle and/or at least one earlobe, wherein the nerve stimulation device (100) comprises the following features:
• a needle device (105) according to any one of claims 1 to 4; and
• a pulse generator (200), which is separable from the needle device (105), for generating electrical pulses, wherein the pulse generator (200) is or can be coupled to the needle device (105) for delivering at least one electrical pulse to the auricle and/or the earlobe.

6. The nerve stimulation device (100) according to claim 5, wherein the pulse generator (200) is configured to deliver electrical pulses within a current range of between 0.05 milliamperes and 1.2 milliamperes.

7. The nerve stimulation device (100) according to any one of the preceding claims 5 or 6, wherein the pulse generator (200) is configured to deliver electrical pulses within a tolerance range around a frequency value of 1 hertz.

8. The nerve stimulation device (100) according to any one of the preceding claims 5 to 7, wherein the pulse generator (200) comprises at least one retaining element (205, 210) for securing the pulse generator (200) to the ear.

9. The nerve stimulation device (100) according to any one of the preceding claims 5 to 8, wherein the pulse generator (200) is rechargeable with electrical energy.

10. The nerve stimulation device (100) according to any one of the preceding claims 5 to 9, wherein the pulse generator (200) is configured to be controlled or controllable using a computing unit, and/or wherein a control program of the pulse generator (200) is modifiable.

11. The nerve stimulation device (100) according to any one of the preceding claims 5 to 10, wherein the pulse generator (200) is configured to perform stimulation of at least one ear nerve at the outer ear and/or stimulation of the vagus nerve.

12. The nerve stimulation device (100) according to any one of the preceding claims 5 to 11, wherein the pulse generator (200) is configured to be attached behind the auricle, and/or wherein the nerve stimulation device (100) is additionally equipped with a loudspeaker, wherein, in particular, the loudspeaker is attached to or arranged in the needle device (105) and/or the pulse generator (200), and/or wherein the nerve stimulation device (100) is additionally equipped with a loudspeaker and/or supplemented by a headphone arranged in an ear.

## Revendications

1. Dispositif à aiguilles (105), conçu pour être placé ou susceptible d'être placé sur le pavillon de l'oreille et/ou le lobe de l'oreille, le dispositif à aiguilles (105) comprenant un élément de préhension (400) et/ou un élément de positionnement (400) destiné à positionner le dispositif à aiguilles (105) sur le pavillon de l'oreille et/ou le lobe de l'oreille, le dispositif à aiguilles (105) comprenant une zone de rupture prédéterminée (600) permettant de séparer l'élément de préhension (400) du dispositif à aiguilles (105), et/ou l'élément de positionnement (400) du dispositif à aiguilles (105) étant réalisé sous la forme d'une pince et/ou d'un clip, et/ou l'élément de positionnement (400) du dispositif à aiguilles (105) comprenant un élément de fixation à encliquetage et/ou un élément de fixation à clic et/ou un élément de fixation magnétique permettant de séparer sans destruction les aiguilles (700) du dispositif à aiguilles (105) de l'élément de positionnement (400).

2. Dispositif à aiguilles (105) selon la revendication 1, le dispositif à aiguilles (105) étant relié ou susceptible d'être relié de manière amovible à un générateur d'impulsions (200) au moyen d'au moins un contact magnétique (605), le générateur d'impulsions (200) étant ou pouvant être couplé au dispositif à aiguilles (105) afin de délivrer au moins une impulsion électrique au pavillon de l'oreille et/ou au lobe de l'oreille, et/ou le dispositif à aiguilles (105) étant relié ou susceptible d'être relié de manière amovible au générateur d'impulsions (200) au moyen d'au moins une pince ou d'une pince à épiler.

3. Dispositif à aiguilles (105) selon l'une quelconque des revendications précédentes, le dispositif à aiguilles (105) étant réalisé sous la forme d'au moins une aiguille-électrode et/ou d'au moins une micro-aiguille et/ou d'un élément à aiguilles comprenant au moins deux aiguilles, en particulier l'au moins une aiguille-électrode et/ou l'au moins une micro-aiguille étant réalisée en un matériau biocompatible, notamment en un métal biocompatible.

4. Dispositif à aiguilles (105) selon l'une quelconque des revendications précédentes, le dispositif à aiguilles (105) comprenant au moins un élément adhésif (405) destiné à fixer le dispositif à aiguilles (105) au pavillon de l'oreille et/ou au lobe de l'oreille.

5. Dispositif de stimulation nerveuse (100) destiné à stimuler électriquement au moins un nerf d'au moins un pavillon de l'oreille et/ou d'au moins un lobe de l'oreille, le dispositif de stimulation nerveuse (100) présentant les caractéristiques suivantes :
• un dispositif à aiguilles (105) selon l'une quelconque des revendications 1 à 4; et
• un générateur d'impulsions (200), séparable du dispositif à aiguilles (105), destiné à générer des impulsions électriques, le générateur d'impulsions (200) étant ou pouvant être couplé au dispositif à aiguilles (105) afin de délivrer au moins une impulsion électrique au pavillon de l'oreille et/ou au lobe de l'oreille.

6. Dispositif de stimulation nerveuse (100) selon la revendication 5, le générateur d'impulsions (200) étant conçu pour délivrer des impulsions électriques dans une plage d'intensité du courant comprise entre 0,05 milliampère et 1,2 milliampère.

7. Dispositif de stimulation nerveuse (100) selon l'une quelconque des revendications précédentes 5 ou 6, le générateur d'impulsions (200) étant conçu pour délivrer des impulsions électriques dans une plage de tolérance autour d'une valeur de fréquence de 1 hertz.

8. Dispositif de stimulation nerveuse (100) selon l'une quelconque des revendications précédentes 5 à 7, le générateur d'impulsions (200) comprenant au moins un élément de maintien (205, 210) destiné à fixer le générateur d'impulsions (200) à l'oreille.

9. Dispositif de stimulation nerveuse (100) selon l'une quelconque des revendications précédentes 5 à 8, le générateur d'impulsions (200) étant rechargeable en énergie électrique.

10. Dispositif de stimulation nerveuse (100) selon l'une quelconque des revendications précédentes 5 à 9, le générateur d'impulsions (200) étant conçu pour être commandé ou susceptible d'être commandé au moyen d'une unité de calcul, et/ou un programme de commande du générateur d'impulsions (200) étant modifiable.

11. Dispositif de stimulation nerveuse (100) selon l'une quelconque des revendications précédentes 5 à 10, le générateur d'impulsions (200) étant conçu pour effectuer une stimulation d'au moins un nerf auriculaire au niveau de l'oreille externe et/ou une stimulation du nerf vague.

12. Dispositif de stimulation nerveuse (100) selon l'une quelconque des revendications précédentes 5 à 11, le générateur d'impulsions (200) étant conçu pour être fixé derrière le pavillon de l'oreille, et/ou le dispositif de stimulation nerveuse (100) étant en outre équipé d'un haut-parleur, ce haut-parleur étant notamment fixé sur ou disposé dans le dispositif à aiguilles (105) et/ou le générateur d'impulsions (200), et/ou le dispositif de stimulation nerveuse (100) étant en outre équipé d'un haut-parleur et/ou complété par un écouteur disposé dans une oreille.
